(19) Europäisches Patentamt
European Patent Office
Office européen des brevets

(11) **EP 4 650 359 A1**

(12) **EUROPEAN PATENT APPLICATION**
published in accordance with Art. 153(4) EPC


(43) Date of publication:
**19.11.2025 Bulletin 2025/47**

(21) Application number: **22952411.1**

(22) Date of filing: **28.07.2022**

(51) International Patent Classification (IPC):
***C07J 43/00*** *(2006.01)* ***C07J 7/00*** *(2006.01)*
***A61K 31/57*** *(2006.01)* ***A61P 25/28*** *(2006.01)*

(52) Cooperative Patent Classification (CPC):
**A61K 31/58; A61K 31/57; A61P 25/24; A61P 25/28; C07J 43/003**

(86) International application number:
**PCT/CN2022/108717**

(87) International publication number:
**WO 2024/020953 (01.02.2024 Gazette 2024/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**



(71) Applicant: **Hunan Kyf Pharmaceutical. Co., Ltd.**
**Jinshi, Hunan 415400 (CN)**

(72) Inventors:
- **LIU, Xirong**
  **Jinshi, Hunan 415499 (CN)**
- **JIN, Zhizhong**
  **Jinshi, Hunan 415499 (CN)**
- **TANG, Jie**
  **Jinshi, Hunan 415499 (CN)**
- **HE, Qun**
  **Jinshi, Hunan 415499 (CN)**
- **ZENG, Chunling**
  **Jinshi, Hunan 415499 (CN)**
- **CHEN, Zhou**
  **Jinshi, Hunan 415499 (CN)**
- **LUO, Guifang**
  **Jinshi, Hunan 415499 (CN)**
- **LI, Long**
  **Shanghai 201609 (CN)**

(74) Representative: **Vial, Lionel et al**
**Cabinet Lionel Vial**
**6 rue de Vaugondran**
**91190 Gif-sur-Yvette (FR)**


# (54) STEROID COMPOUND FOR TREATING CENTRAL NERVOUS SYSTEM DISEASE, METHOD FOR PREPARING SAME, AND USE AND PHARMACEUTICAL COMPOSITION THEREOF


(57) Provided are a steroid compound for treating a central nervous system disease, a method for preparing same, and use and a pharmaceutical composition thereof. The steroid compound has a 9β,10α structure and has the following structural formula (I). The steroid compound acts as a GABA regulator, prolongs the opening time of a chloride ion channel activated by GABA, adjusts the excitability of the central nervous system (CNS), and treats and prevents CNS-related diseases. Besides, the 9β,10α steroid compound of the present invention has a sedative effect and can be used as a sedative.


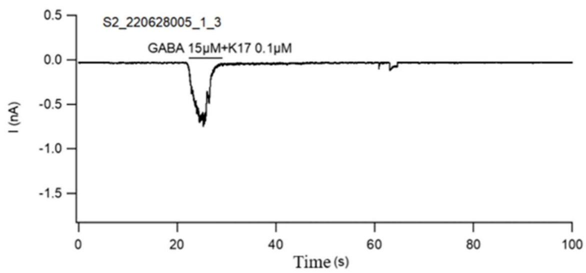

Fig. 2

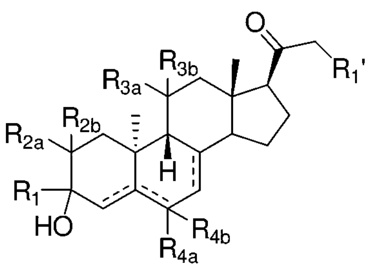

EP 4 650 359 A1

Processed by Luminess, 75001 PARIS (FR)

## Description

### FIELD

The present application pertains to the technical field of pharmaceuticals, and specifically relates to steroid compounds for treating central nervous system diseases, their preparation methods, uses and pharmaceutical compositions.

### BACKGROUND

Brain excitability is defined as the arousal level of animals and is a continuum ranging from coma to convulsion, which is regulated by various neurotransmitters. GABA (γ-aminobutyric acid) is the major inhibitory neurotransmitter in the central nervous system of vertebrates. Up to 40% of the neurons in the brain utilize GABA as a neurotransmitter. GABA receptors are a class of receptors within cells that respond to the neurotransmitter GABA. GABA receptors have three subtypes, namely GABAA receptor, GABAB receptor and GABAC receptor.
The professional term "neurosteroids" was put forward by E. Baulieu from France in 1981. Neurosteroids can regulate neurological functions by binding to different neural receptors (such as GABAA receptors), alter the ability of brain excitability, and then treat or alleviate diseases related to the CNS (central nervous system). Neurosteroids produce various physiological and pathological effects through binding to receptors, mainly acting through ligand-gated ion channels. The GABAA receptor belongs to the ligand-gated ion channel. Once activated, it opens the chloride ion channel, triggers the influx of chloride ions, causes cell hyperpolarization, and thus exerts an inhibitory effect on neurotransmission and reduces central excitability. The higher the concentration of chloride ions in neurons is, the lower the brain excitability (arousal level) will be.

Known neurosteroid drugs include allopregnanolone/Brexanolone, pregnenolone (PREG), etc. Among them, allopregnanolone is a highly effective synaptic and extrasynaptic GABAA receptor positive allosteric modulator, which can prolong the opening time of the chloride ion channel caused by GABA.
However, these neurosteroid drugs have some problems in terms of the extensiveness of indications, drug efficacy, toxic and side effects, bioavailability, metabolic stability, etc. There is a need for new and improved neuroactive steroids to be used as modulators of brain excitability and drugs for the prevention and treatment of CNS-related diseases.

### SUMMARY

The present invention provides a steroid compound or its pharmaceutically - acceptable salt. The steroid compound has a 9β, 10α structure and the following structural formula:

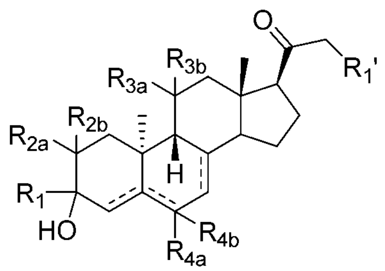

Formula 1

wherein $R_1$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, or $-CH_2OR_{1a}$, where $R_{1a}$ is selected from substituted or unsubstituted $C_{1\sim6}$ alkyl, or substituted or unsubstituted $C_{2\sim6}$ alkenyl;
wherein $R_{2a}$ and $R_{2b}$ are each independently selected from H, halogen, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group or $OR_{2c}$, where $R_{2c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group;
$R_{3a}$ and $R_{3b}$ are each independently selected from H or $OR_{3c}$, where $R_{3c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group, or $R_{3a}$ and $R_{3b}$ combine to form = O;

$R_{4a}$ and $R_{4b}$ are each independently selected from H, halogen, substituted or unsubstituted $C_{1\sim6}$ alkyl;
$R_1$' is selected from H, or substituted or unsubstituted heteroaryl;
- - - - - - represents a single bond or a double bond;
When one of - - - - - - is a double bond, the adjacent - - - - - - is a single bond.

In some embodiments of the present invention, the steroid compound has the following structural formula:

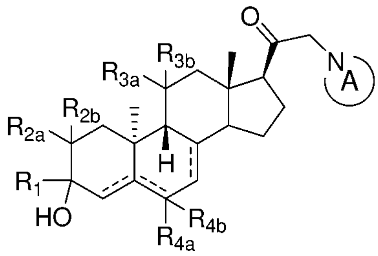

among which, ring A is a substituted or unsubstituted heteroaryl group, the heteroatom is N, and the number of heteroatom N is 1~4.

In some embodiments of the present invention, the ring A is selected from the following groups

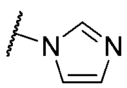 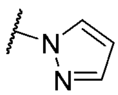 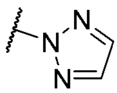 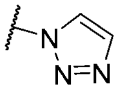

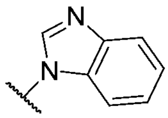 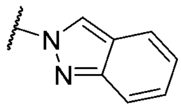 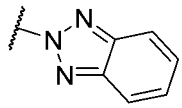 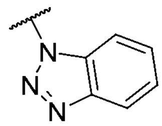

The above groups are either unsubstituted or substituted by groups selected from the following: H, halogen, $-NO_2$, -CN, -OR', $-N(R')_2$, -C(=O)R', -C(=O)OR', -OC(=O)R' or -OC(=O)OR', wherein R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl group, or substituted or unsubstituted heteroaryl group.

In some embodiments of the invention, the steroid compound has the following structural formula

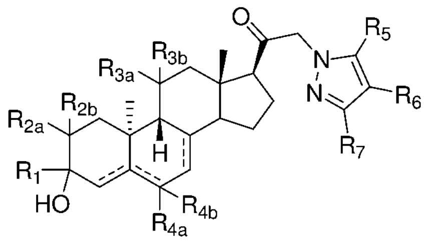

wherein $R_5$, $R_6$ and $R_7$ are each independently selected from H, halogen, $-NO_2$, -CN, -OR', $-N(R')_2$, -C(=O)R', -C(=O)OR', -OC(=O)R' or -OC(=O)OR', where R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl, or substituted or unsubstituted heteroaryl.

In some embodiments of the present invention, the steroid compound is selected from the following structural formula

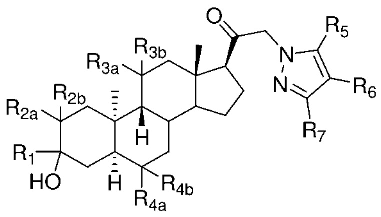 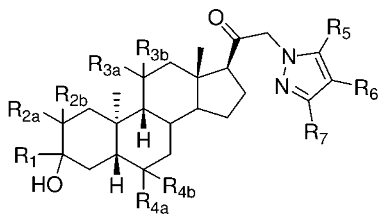

In some embodiments of the present invention, the steroid compound is selected from the following structural formula

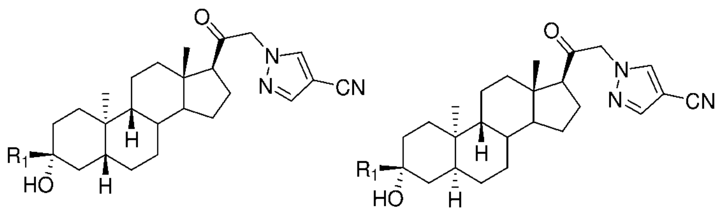

In some embodiments of the present invention, the steroid compound is selected from the following structural formula

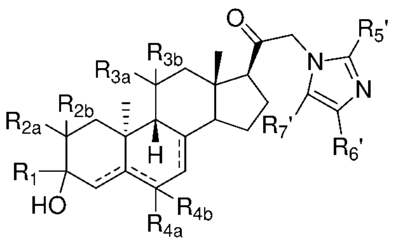

wherein $R_5$', $R_6$' and $R_7$' are each independently selected from H, halogen, $-NO_2$, -CN, -OR', $-N(R')_2$, -C(=O)R', -C(=O)OR', -OC(=O)R' or -OC(=O)OR', wherein R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl, or substituted or unsubstituted heteroaryl.

In some embodiments of the present invention, the steroid compound is selected from the following structural formula

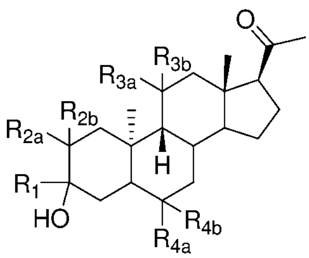

The present invention provides a method for preparing a steroid compound having the following structural formula:

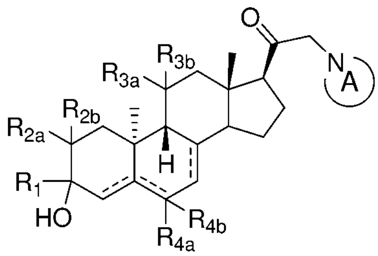

The steroid compound is prepared by nucleophilic substitution reaction of the compound of the following formula

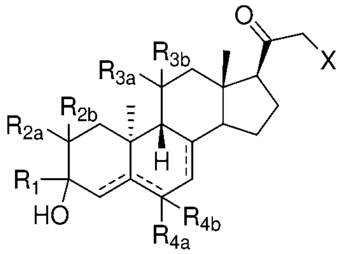

wherein X is a halogen.

The present invention provides a method for preparing a steroid compound having the following structural formula:

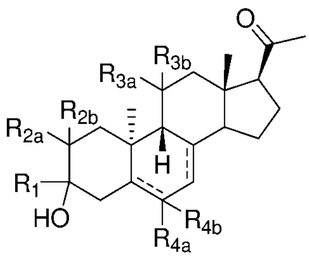

The steroid compound is prepared through a reduction reaction and/or an addition reaction from dydrogesterone.

The present invention also provides an intermediate compound for preparing the steroid compound of the present invention, and the intermediate compound has the following structural formula:

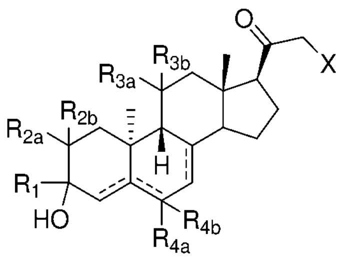

wherein X is a halogen.

The present invention also provides the use of the steroid compound of the present invention or its pharmaceutically acceptable salt in the preparation of drugs for treating central nervous system-related disorders.

In some embodiments of the present invention, the central nervous system-related disorders are selected from: sleep disorders, mood disorders, schizophrenia spectrum disorders, spastic disorders, memory disorders and/or cognitive disorders, movement disorders, personality disorders, autism spectrum disorders, pain, and traumatic brain injury.

In some embodiments of the present invention, the central nervous system-related disorders are selected from: insomnia, depression, anxiety, epilepsy, dementia, neuropathic pain or tremor.

In some embodiments of the present invention, the dementia is Alzheimer's disease.

The present invention also provides a pharmaceutical composition comprising the steroid compound or its

pharmaceutically acceptable salt according to the present invention, and pharmaceutically acceptable excipients.

Compared with the prior art, the present invention obtains at least the following beneficial technical effects, for example:

The 9β,10α steroid compound of the present invention acts as a GABA modulator, prolongs the opening time of the chloride ion channel activated by GABA, regulates the excitability of the central nervous system (CNS), and treats and prevents CNS-related disorders. Moreover, the 9β,10α steroid compound of the present invention has a sedative effect and can be used as a sedative, having a stronger sedative effect on male animals and men.

Different from traditional steroid compounds, the methyl group connected to the C-10 position of the compound of the present invention has an α configuration (while the H at the 9 position has a β configuration), and the overall conformation is changed. Compared with the β configuration at the C-10 position, the compound of the present invention has better efficacy, pharmacokinetic properties, bioavailability, stability and safety.

Compared with traditional steroid compounds with the β configuration at the C-10 position, the 9β,10α steroid compound of the present invention has stronger binding specificity and higher activity with the GABAA receptor; it has more selectivity in binding with the GABAA receptor, reduces the binding with non-target receptors, such as hormone receptors, shows the action of steroid hormones less, and has fewer side effects.

Meanwhile, compared with compounds with the β configuration at the C-10 position, the 9β,10α steroid compound of the present invention is less sensitive to enzymes in the body, the reactivity with enzymes in the body is greatly reduced, it is not easy to be degraded under the action of enzymes, the metabolism in the body is slower, and the duration of drug efficacy is longer.

In addition, the drug composition prepared by using the 9β,10α steroid compound of the present invention has a more flexible administration mode, and it can be taken orally and is convenient to use.

## BRIEF DESCRIPTION OF DRAWINGS

Figure 1 shows the GABA receptor current-time detection spectrogram of GABA at 15 μM.
Figure 2 shows the GABA receptor current-time detection spectrogram of GABA at 15 μM + 0.1 μM of TM 17.
Figure 3 shows the GABA receptor current-time detection spectrogram of GABA at 15 μM + 1 μM of TM 17.
Figure 4 shows the calculation result of the EC50 of TM 17.
Figure 5 shows the calculation result of the EC50 of TM 18.

## DETAILED DESCRIPTION

To make the objectives, technical solutions and advantages of the present invention clearer, the present invention will be further described in detail below. However, it should be understood that the description herein is only used to explain the present invention and is not intended to limit the scope of the present invention.

Unless otherwise defined, all the technical terms and scientific terms used herein have the same meanings as those commonly understood by the technicians in the technical field of the present invention. The terms used in the specification of the present invention are only for the purpose of describing specific embodiments and are not intended to limit the present invention. The reagents and instruments used herein are all commercially available, and the characterization methods involved can refer to the relevant descriptions in the prior art, and will not be repeated here.

### Terms

In the present invention, the term "alkyl" refers to a straight-chain or branched-chain saturated aliphatic hydrocarbon group. In some embodiments, the alkyl group has 1 - 8 carbon atoms (denoted as $C_{1-8}$ alkyl). In some embodiments, the alkyl group has 1 - 6 carbon atoms ($C_{1-6}$ alkyl). In some embodiments, the alkyl group has 1 - 3 carbon atoms ($C_{1-3}$ alkyl). Non-limiting examples of $C_{1-6}$ alkyl include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, and various branched isomers thereof, etc. The alkyl group may be substituted or unsubstituted. When substituted, the substituents may be substituted at any available attachment points, and the substituents are preferably one or more of the following groups, which are independently selected from alkyl, alkenyl, alkynyl, carbocyclic group, alkoxy, halogen, hydroxyl, oxo group, amino, amine group, acyl group, acyloxy or ester group.

The term "alkenyl" refers to an alkyl group as defined above, which consists of at least two carbon atoms and at least one carbon-carbon double bond. In some embodiments, the alkenyl group is $C_{2-8}$ alkenyl. In some embodiments, the alkenyl group is $C_{2-6}$ alkenyl. In some embodiments, the alkenyl group is $C_{2-4}$ alkenyl. The carbon-carbon double bond may be internal (for example, in 2-butenyl) or terminal (for example, in 1-butenyl). Non-limiting examples of $C_{2-6}$ alkenyl

include vinyl, 1-propenyl, 2-propenyl, 1-, 2- or 3-butenyl, etc. The alkenyl group may be substituted or unsubstituted. When substituted, the substituents are preferably one or more of the following groups, which are independently selected from alkyl, haloalkyl, alkenyl, alkynyl, carbocyclic group, alkoxy, halogen, hydroxyl, oxo group, amino, amine group, acyl group, acyloxy or ester group.

The term "alkynyl" refers to an alkyl group as defined above, which consists of at least two carbon atoms and at least one carbon-carbon triple bond. In some embodiments, the alkynyl group is $C_{2-8}$ alkynyl. In some embodiments, the alkynyl group is $C_{2-6}$ alkynyl. In some embodiments, the alkynyl group is $C_{2-4}$ alkynyl. Non-limiting examples include ethynyl, 1-propynyl, 2-propynyl, 1-butynyl, etc. The alkynyl group may be optionally substituted or unsubstituted. When substituted, the substituents are preferably one or more of the following groups, which are independently selected from alkyl, haloalkyl, alkenyl, alkynyl, carbocyclic group, alkoxy, halogen, hydroxyl, oxo group, amino, amine group, acyl group, acyloxy or ester group.

The term "carbocyclic group" refers to a saturated or partially unsaturated all-carbon non-aromatic cyclic hydrocarbon group. A saturated carbocyclic group is called a "cycloalkyl". In some embodiments, the carbocyclic group contains 3 - 6 ring carbon atoms. In some embodiments, the carbocyclic group contains 5 or 6 ring carbon atoms. Non-limiting examples of $C_{3-6}$ carbocyclic group include cyclopropyl, cyclopentyl, cyclopentenyl, cyclohexyl, cyclohexenyl, cyclohexadienyl, etc. The carbocyclic group may be optionally substituted or unsubstituted. When substituted, the substituents are preferably one or more of the following groups, which are independently selected from alkyl, haloalkyl, alkenyl, alkynyl, carbocyclic group, alkoxy, halogen, hydroxyl, oxo group, amino, amine group, acyl group, acyloxy or ester group.

The term "heterocyclic group" refers to a saturated or partially unsaturated cyclic hydrocarbon substituent in which one or more ring atoms are heteroatoms selected from nitrogen or oxygen, and the remaining ring atoms are carbon. In some embodiments, the heterocyclic group contains 3 to 12 ring atoms, among which 1 to 4 are heteroatoms. Non-limiting examples of heterocyclic group include pyrrolidinyl, imidazolidinyl, tetrahydrofuranyl, dihydroimidazolyl, dihydrofuranyl, piperidinyl, piperazinyl, pyranyl, etc. The heterocyclic group may be substituted or unsubstituted. When substituted, the substituents are preferably one or more of the following groups, which are independently selected from alkyl, haloalkyl, alkoxy, halogen, hydroxyl or amino.

The term "aryl" refers to an all-carbon monocyclic or fused polycyclic (i.e., rings sharing adjacent pairs of carbon atoms) group having a conjugated π-electron system. In some embodiments, the aryl group has 6 - 10 ring carbon atoms, such as phenyl, 1-naphthyl, 2-naphthyl, etc. The aryl group may be substituted or unsubstituted. When substituted, the substituents are preferably one or more of the following groups, which are independently selected from alkyl, haloalkyl, alkoxy, halogen, hydroxyl or amino.

The term "heteroaryl" refers to a heteroaromatic system containing 1 to 4 heteroatoms and 5 to 14 ring atoms, where the heteroatoms are selected from oxygen and nitrogen, preferably nitrogen. The heteroaryl group is preferably 5 to 10 membered, such as imidazolyl, pyrazolyl, pyrrolyl, triazolyl, tetrazolyl, pyridyl, pyrazinyl, etc. The heteroaryl ring may be fused to an aryl ring, where the ring connected to the parent structure is the heteroaryl ring, and non-limiting examples thereof include:

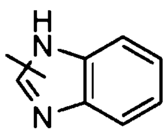 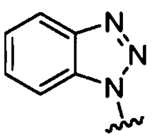 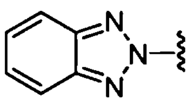 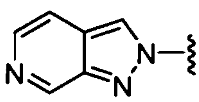 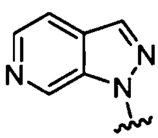

The heteroaryl group may be substituted or unsubstituted. When substituted, the substituents are preferably one or more of the following groups, which are independently selected from alkyl, haloalkyl, halogen or amino.

"Alkoxy" refers to -O-(alkyl) and -O-(cycloalkyl), where the definitions of alkyl and carbocyclic group are as described above. Non-limiting examples of alkoxy include: methoxy, ethoxy, propoxy, butoxy, cyclopentyloxy, cyclohexyloxy, etc.

"Acyl" refers to the group $-C(O)R^a$, where Ra is hydrogen, alkyl, alkenyl, alkynyl, carbocyclic group or aryl as defined herein. Non-limiting examples include: acetyl ($-C(O)CH_3$), cyclohexylcarbonyl, benzoyl (-C(O)Ph), etc.

"Acyloxy" refers to the group $-OC(O)R^b$, where $R^b$ is hydrogen, alkyl, alkenyl, alkynyl, carbocyclic group or aryl as defined herein.

"Ester group" refers to the group $-COOR^c$, where $R^c$ is alkyl, alkenyl, alkynyl, carbocyclic group or aryl as defined herein.

"Amine group" refers to the group -NR'R", where R', R" are hydrogen, alkyl as defined herein, and at least one of R', R" is not hydrogen.

"Haloalkyl" refers to an alkyl group substituted by one or more halogens, where the alkyl group is as defined above.

"Halogen" refers to fluorine, chlorine, bromine or iodine.

"Hydroxyl" refers to the -OH group.
"Oxo group" refers to =O.
"Amino" refers to $-NH_2$.
"Carboxyl" refers to -C(O)OH.
When a range of values is enumerated, it is intended to include each value and sub-range within the said range. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.
"Plurality" refers to more than two, such as 2-5, 2-3, 2, 3, 4 or 5, etc.
Different expressions such as "X is selected from A, B or C", "X is selected from A, B and C", "X is A, B or C", "X is A, B and C" all convey the same meaning, that is, X can be any one or several of A, B and C.
"Optional" or "optionally" means that the event or circumstance described subsequently may but need not occur, and the description includes both the cases where the event or circumstance occurs and where it does not occur. For example, "aryl optionally substituted by alkyl" means that the alkyl may but does not necessarily exist, and the description includes both the case where the aryl is substituted by alkyl and the case where the aryl is not substituted by alkyl.
"Substituted" means that one or more hydrogen atoms in a group are independently substituted by a corresponding number of substituents. It goes without saying that the substituents are only in their possible chemical positions, and those skilled in the art can determine (through experiment or theory) possible or impossible substitutions without undue effort.
"Pharmaceutically acceptable salt" refers to a salt of a compound of the present invention that is pharmaceutically acceptable and has the desired pharmacological activity of the parent compound. Specifically, such salts are non-toxic and may be organic or inorganic acid addition salts and base addition salts.

<u>**The steroid compounds of the present invention**</u>

For the convenience of understanding this article, the carbon atom numbering of steroid compounds is shown below. For example, the 10th carbon atom can be denoted as the C-10 position. The four rings are denoted as A, B, C and D from left to right respectively.

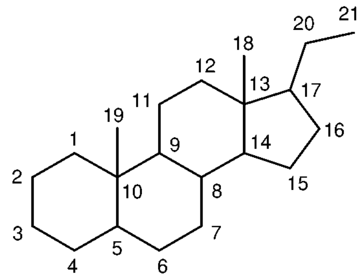

The compounds described in this article can exist in various isomeric forms, such as enantiomers and/or diastereomers. For example, the compounds described herein can be individual enantiomers, diastereomers or geometric isomers, or can be in the form of mixtures of stereoisomers, including racemic mixtures and mixtures enriched in one or more stereoisomers. Isomers can be separated from mixtures by methods known to those skilled in the art, which include chiral high-performance liquid chromatography (HPLC) as well as the formation and crystallization of chiral salts; or preferred isomers can be prepared by asymmetric synthesis. The present invention further includes the compounds described herein either as individual isomers substantially free of other isomers or as mixtures of multiple isomers.
The configurations of the general formula compounds (including at least two compounds) with an $\alpha$-methyl group at the C-10 position and specific compounds (compounds in the examples, including intermediates) involved in the present invention are as follows:

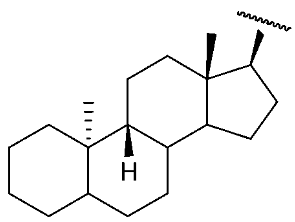

In addition to the groups whose configurations have been indicated in the above formula, other groups or

hydrogen atoms connected to the steroid ring can have either α or β configurations. The configurations of TM17-20 and TM25-28 in the examples are as follows:

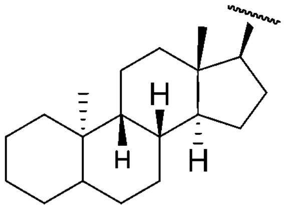

The 9β,10α steroid compounds of the present invention have stereoconformations different from those of traditional steroid compounds. Taking the 5α series as an example, the A-B ring conformations of the 9β,10α steroid compounds of the present invention and the traditional 9α,10β steroid compounds are shown below respectively.

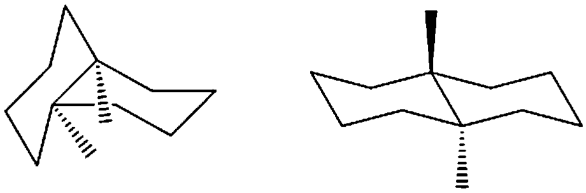

In an embodiment of the present invention, it provides a steroid compound or a pharmaceutically acceptable salt thereof. The steroid compound has a 9β, 10α structure and has the following structural formula:

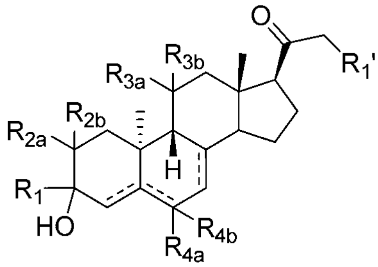

Formula I

wherein $R_1$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, or $-CH_2OR_{1a}$, where $R_{1a}$ is selected from substituted or unsubstituted $C_{1\sim6}$ alkyl, or substituted or unsubstituted $C_{2\sim6}$ alkenyl;

wherein $R_{2a}$ and $R_{2b}$ are each independently selected from H, halogen, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group or $OR_{2c}$, where $R_{2c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group;

$R_{3a}$ and $R_{3b}$ are each independently selected from H or $OR_{3c}$, where $R_{3c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group, or $R_{3a}$ and $R_{3b}$ combine to form =O;

$R_{4a}$ and $R_{4b}$ are each independently selected from H, halogen, substituted or unsubstituted $C_{1\sim6}$ alkyl;

$R_1$' is selected from H, or substituted or unsubstituted heteroaryl;

------ represents a single bond or a double bond;

when one of ------ is a double bond, the adjacent ------ is a single bond.

In some embodiments, the $R_1$ is selected from H, $C_{1\sim3}$ alkyl, $C_{1\sim3}$ haloalkyl or $-CH_2OR_{1a}$, wherein $R_{1a}$ is selected from $C_{1\sim3}$ alkyl.

In some embodiments, the $R_1$ is selected from H.

In some embodiments, the $R_1$ is selected from methyl.

In some embodiments, the $R_1$ is selected from $-CH_2OCH_3$.

In some embodiments, the configuration of $R_1$ is of the $\alpha$ form or the $\beta$ form. Correspondingly, the configuration of the -OH at the C-3 position is $\beta$-OH or $\alpha$-OH.

In some embodiments, the configuration of $R_1$ is of the $\beta$ form and the C-3 position is $\alpha$-OH.

In some embodiments, $R_{2a}$, $R_{2b}$, $R_{3a}$, $R_{3b}$, $R_{4a}$ and $R_{4b}$ are all H.

In some embodiments, - - - - - - is a single bond, the C-8 position is $\beta$-H and the C-14 position is $\alpha$-H.

First aspect ($R_1$' is a substituted or unsubstituted heteroaryl)

In some embodiments, when $R_1$' is selected from substituted or unsubstituted heteroaryl, the heteroatom therein is N.

In some embodiments, the steroid compound has the following structural formula:

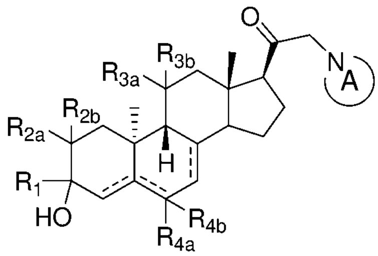

wherein ring A is a substituted or unsubstituted heteroaryl group, the heteroatom is N, and the number of heteroatom N is 1 to 4.

In some embodiments, the ring A is a five-membered ring, and the number of heteroatom N is 2 or 3.

In some embodiments, ring A is selected from the following groups.

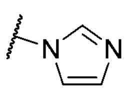 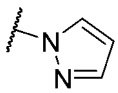 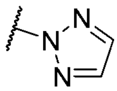 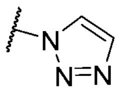

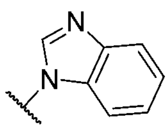 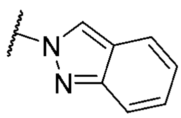 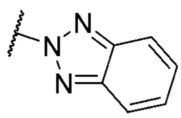 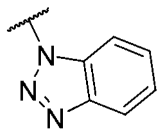

The above groups are either unsubstituted or substituted by groups selected from the following: H, halogen, $-NO_2$, -CN, -OR', $-N(R')_2$, -C(=O)R', -C(=O)OR', -OC(=O)R' or -OC(=O)OR', where R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl, or substituted or unsubstituted heteroaryl.

(1.1)

In some embodiments, the steroid compound has the following structural formula:

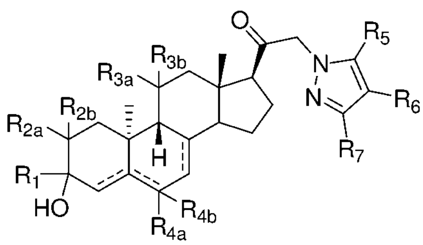

wherein $R_5$, $R_6$ and $R_7$ are each independently selected from H, halogen, $-NO_2$, -CN, -OR', $-N(R')_2$, -C(=O)R', -C(=O)OR', -OC(=O)R' or -OC(=O)OR', where R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl, or substituted or unsubstituted heteroaryl.

In some embodiments, one of $R_5$, $R_6$ and $R_7$ is -CN, and the others are H.

In some embodiments, the $R_1$ is selected from H, $C_{1\sim3}$ alkyl or $C_{1\sim3}$ haloalkyl, and $R_{2a}$, $R_{2b}$, $R_{3a}$, $R_{3b}$, $R_{4a}$ and $R_{4b}$ are all H.

In some embodiments, the steroid compound is selected from the following structural formulas:

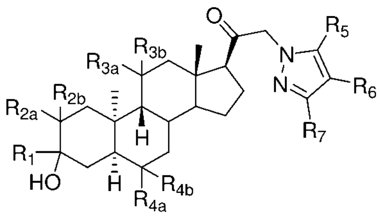

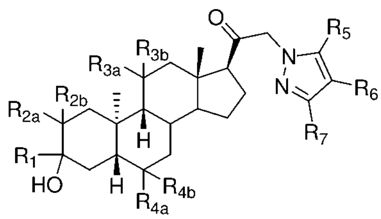

In some embodiments, the steroid compound is selected from the following structural formula:

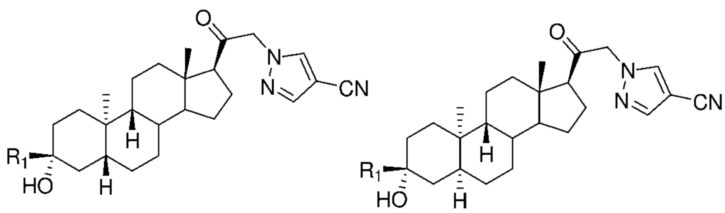

In some embodiments, the $R_1$ is selected from methyl or ethyl.

(1.2)

In some embodiments, the steroid compound has the following structural formula:

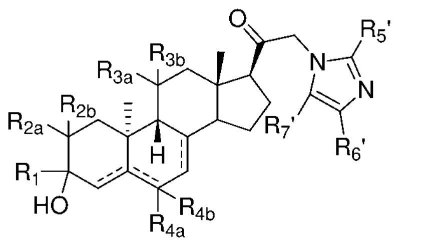

wherein $R_5$', $R_6$' and $R_7$' are each independently selected from H, halogen, $-NO_2$, -CN, -OR', $-N(R')_2$, -C(=O)R', -C(=O)OR', -OC(=O)R' or -OC(=O)OR', where R' is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, substituted or unsubstituted $C_{3\sim6}$ heterocyclic group, substituted or unsubstituted aryl, or substituted or unsubstituted heteroaryl.

In some embodiments, the $R_5$', $R_6$' and $R_7$' are all H.

In some embodiments, the $R_1$ is selected from H, $C_{1-3}$ alkyl or $C_{1-3}$ haloalkyl, and $R_{2a}$, $R_{2b}$, $R_{3a}$, $R_{3b}$, $R_{4a}$ and $R_{4b}$ are all H, and - - - - - - is a single bond.

In some embodiments, the steroid compound is selected from the following structural formulas:

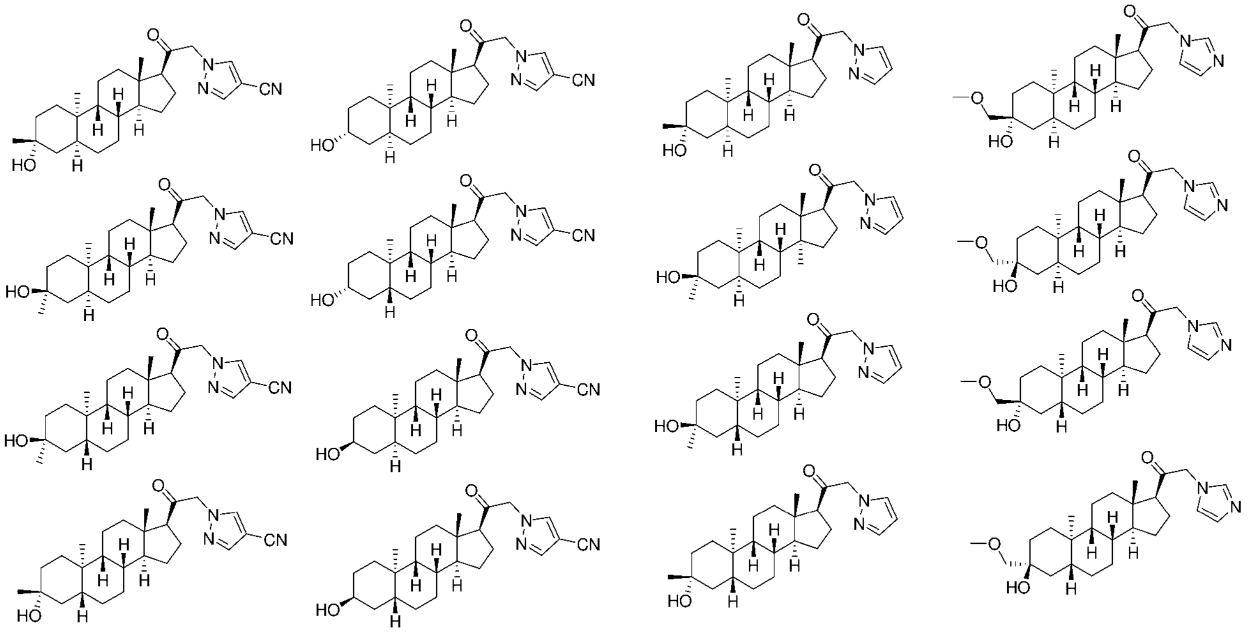

Second aspect ($R_1$' is H)

In some embodiments, the steroid compound has the following structural formula:

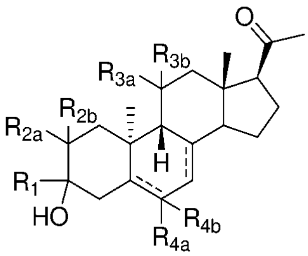

In some embodiments, the steroid compound has the following structural formula:

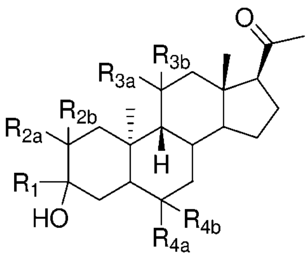

In some embodiments, the steroid compound is selected from the following structural formulas:

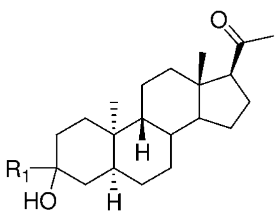

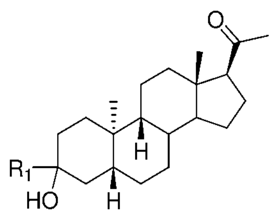

In some embodiments, the steroid compound is selected from the following structural formulas:

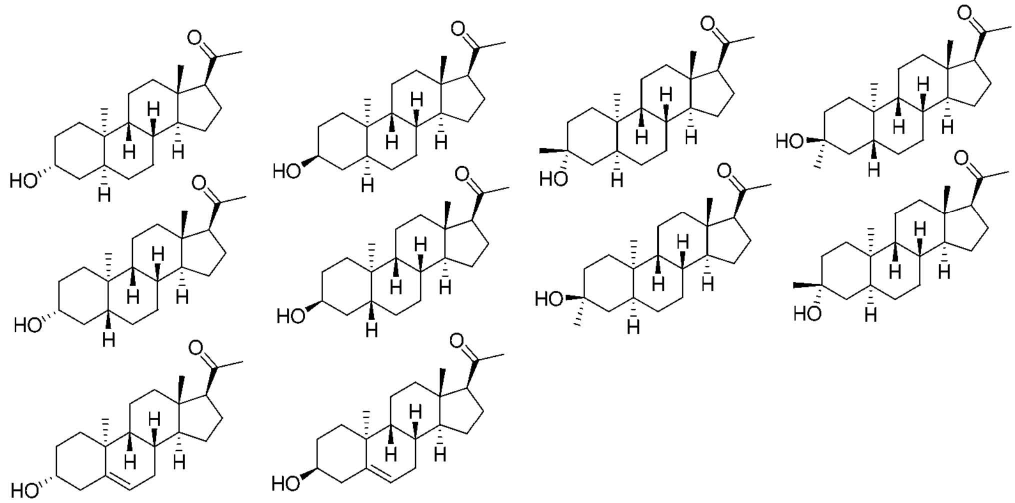

Use **of Steroid Compounds**

The steroid compounds of the present invention or their pharmaceutically acceptable salts can regulate GABA and are used for the treatment and prevention of CNS - related disorders. Exemplary CNS disorders related to GABA regulation include, but are not limited to: sleep disorders [such as insomnia], mood disorders [such as depression, postpartum depression, mental depressive disorders (such as mild depression), bipolar disorders (such as I and/or II), anxiety disorders (such as generalized anxiety disorder (GAD), social anxiety disorder), stress responses, post - traumatic stress disorder (PTSD), obsessive - compulsive disorders (such as obsessive - compulsive disorder (OCD))], schizophrenia - spectrum disorders [such as schizophrenia, schizoaffective disorder], spastic / convulsive disorders [such as epilepsy (such as status epilepticus (SE)), seizures], memory disorders and/or cognitive disorders [such as attention disorders (such as attention - deficit hyperactivity disorder (ADHD)), dementia (such as Alzheimer - type dementia, Lewy - body - type dementia, vascular - type dementia)], movement disorders [such as Huntington's disease, Parkinson's disease], personality disorders [such as antisocial personality disorder, obsessive - compulsive personality disorder], autism - spectrum disorders (ASD) [such as autism, monogenic autism], tremors (such as essential tremor), pain [such as neuropathic pain], traumatic brain injury (TBI).

Clinical depression is also known as major depression, major depressive disorder (MDD), major depressive episode, unipolar depression, unipolar disorder, and recurrent depression, and refers to a mental disorder characterized by a pervasive and persistent low mood, accompanied by low self - esteem and a loss of interest or pleasure in normally enjoyable activities. The symptoms of depression and their remission can be determined by a physician or a psychologist (for example, through a mental status examination).

"Treatment" of any disease or disorder means the improvement of such a disease or disorder (i.e., the arrest (inhibition) of the disease or the reduction of the manifestation, extent, or severity of at least one of its clinical symptoms). In another embodiment, "treatment" means the improvement of at least one physical parameter that may not be discernible by the subject. In yet another embodiment, "treatment" means the physical (e.g., stabilization of discernible symptoms), physiological (e.g., stabilization of physical parameters), or both regulation of a disease or disorder. In an additional embodiment, "treatment" involves slowing the progression of the disease.

"Effective amount" means the amount of a compound that is sufficient to achieve such treatment when administered to a subject for the treatment of a disease.

**Pharmaceutical Compositions**

The present invention provides a pharmaceutical composition comprising the steroid compound according to the present invention or a pharmaceutically acceptable salt thereof, as well as pharmaceutically acceptable excipients.

The dosage forms of the pharmaceutical composition of the present invention include: tablets, capsules, granules, powders, aerosols, dry powder inhalants, sprays, suspensions, solutions, emulsions, injections, and the like. Depending on the characteristics of their respective dosage forms, the routes of administration include oral, sublingual, injection (intravenous/intramuscular), pulmonary/tracheal, and so on.

In some embodiments, the pharmaceutical composition provided by the present invention is an oral solid preparation, preferably tablets. In addition to the active ingredient, this oral solid preparation also contains pharmaceutical excipients. The pharmaceutical excipients mentioned are all conventional pharmaceutical excipients in the art, including fillers (also known as diluents), disintegrants, binders or wetting agents, lubricants (including glidants), and so on. The

amounts of the pharmaceutical excipients can be in accordance with conventional dosages.

The fillers generally include lactose, microcrystalline cellulose, mannitol, pregelatinized starch, starch, sucrose, dextrin, sorbitol, calcium carbonate, calcium bicarbonate, hydroxypropyl methylcellulose, and ethylcellulose, etc. They can be used alone or in combination.

The disintegrants generally include starch, sodium carboxymethylcellulose, calcium carboxymethylcellulose, sodium carboxymethyl starch, croscarmellose sodium, crospovidone, and low-substituted hydroxypropylcellulose, etc. They can be used alone or in combination.

The binders or wetting agents generally include povidone (polyvinylpyrrolidone), hydroxypropyl methylcellulose, hydroxypropylcellulose, ethylcellulose, polyethylene glycol, starch paste, water, and ethanol solutions of various concentrations, etc. They can be used alone or in combination.

The lubricants generally include zinc stearate, magnesium stearate, calcium stearate, sodium stearyl fumarate, talc powder, sucrose fatty acid esters, colloidal silicon dioxide, stearic acid, and solid polyethylene glycol, etc. They can be used alone or in combination.

If necessary, other excipients can also be added to the above composition, such as sweeteners (such as aspartame, stevioside, etc.), colorants (such as yellow iron oxide, red iron oxide, etc.), stabilizers (such as citric acid, lactic acid, malic acid, etc.), pH regulators (such as sodium bicarbonate, fumaric acid, citric acid, etc.).

If necessary, other suitable active ingredients can also be included in the above composition.

The preparation of the above oral solid preparation can be carried out according to the conventional methods for preparing oral solid preparations in the technical field. For example, tablets can be prepared by wet granulation and tableting, dry granulation and tableting, fluidized bed granulation and tableting, direct powder mixing and tableting, and so on. When the oral solid preparation is tablets, they can be further coated as needed to make film-coated tablets or sugar-coated tablets. The coating materials include cellulose-based materials, acrylic resin-based materials, and sugars, such as hydroxypropyl methylcellulose and sucrose, etc. Plasticizers, anti-tacking agents, and opacifying agents can also be added thereto.

The dosage of the above composition is adjusted according to the nature and severity of the patient's condition, the route of administration, and the patient's age, weight, etc.

## Preparation of the Steroid Compounds of the Present Invention

### First Aspect

In the embodiments of the present invention, when the steroid compound has the following structural formula:

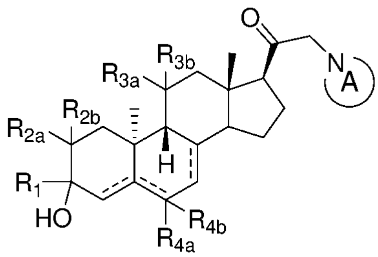

wherein ring A is a substituted or unsubstituted heteroaryl group, the heteroatom is N, and the number of heteroatom N is 1 to 4; the steroid compound is prepared by a nucleophilic substitution reaction of a compound of the following formula:

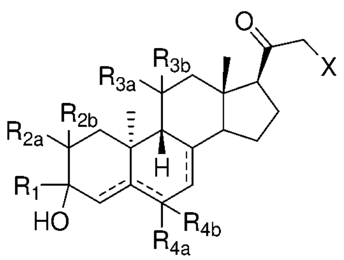

wherein X is a halogen.

Correspondingly, the present invention provides an intermediate compound for preparing the steroid compound, and the intermediate compound has the following structural formula:

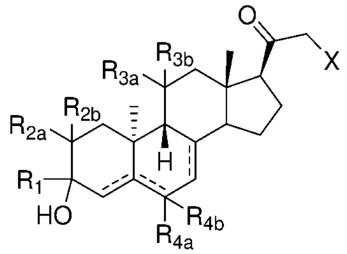

wherein X is a halogen.

Second Aspect

In the embodiments of the present invention, when the steroid compound has the following structural formula:

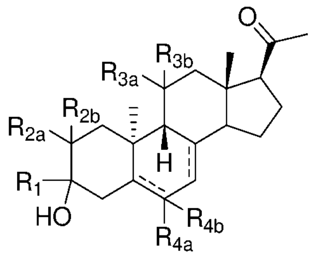

The steroid compound is prepared by subjecting dydrogesterone to a reduction reaction and/or an addition reaction.

The compounds of the present invention can be prepared by various synthetic routes and methods. As an example, some of the compounds of the present invention can be synthesized through Route L1 or Route L2 as follows.

Route L1

Using dydrogesterone as a starting material, TM1 and TM2 are first prepared through the following process route, and then TM9 - TM12 are prepared using TM1 and TM2, and then TM25 - TM28 are prepared from TM9 - TM12.

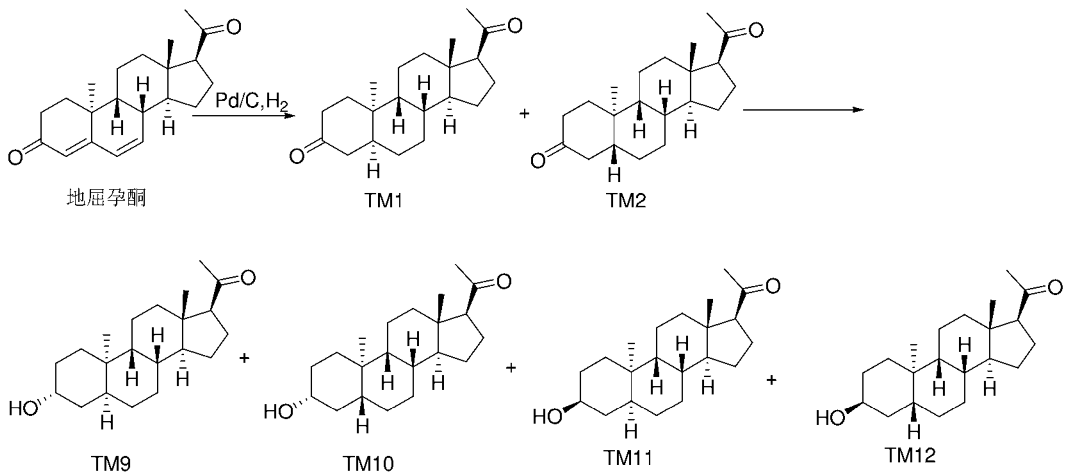


地屈孕酮 TM1 + TM2

TM9 + TM10 + TM11 + TM12

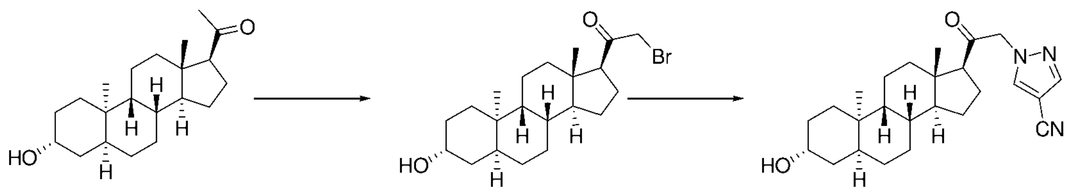

TM9 TM25

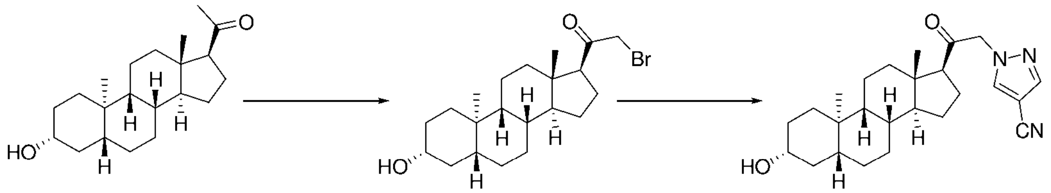

TM10 TM26

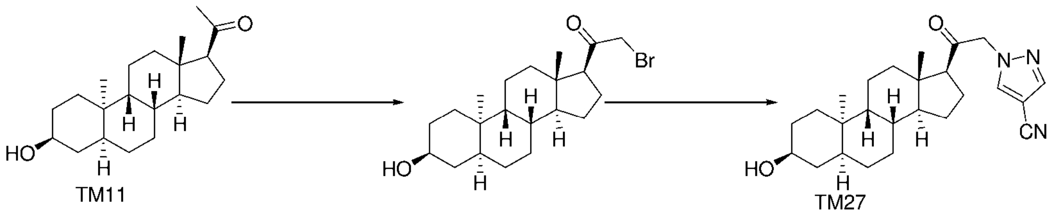

TM11 TM27

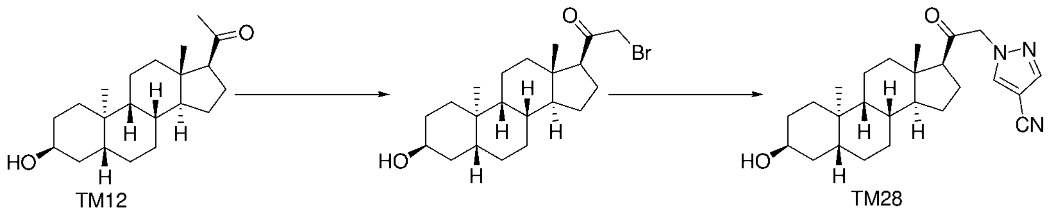

TM12 TM28

Synthesis of TM1 and TM2

Using dydrogesterone as a starting material, TM1 and TM2 are obtained through a catalytic hydrogenation reaction. The reaction catalyst is a metal catalyst such as palladium on carbon, platinum on carbon, etc. The reaction solvent is tetrahydrofuran, ethyl acetate, methanol or a mixed solvent thereof. The reaction temperature ranges from room temperature to 50 °C, and the reaction time is 4-16 h. When methanol is used as the solvent, a by-product of 3-position ketal with low polarity will be generated and needs to be decomposed by adding an acid during post-treatment. The polarities of TM1 and TM2 are very close, and the polarity of TM1 is slightly lower than that of TM2. The reaction solution is filtered to obtain a mixed solution of TM1 and TM2. The product is mainly TM1, and TM1 can be obtained by recrystallization from isopropyl ether, while TM2 can be obtained by column chromatography.

Synthesis of TM9 - TM12

On the basis of preparing TM1 and TM2, the carbonyl group at the 3-position is selectively reduced through the Meerwein-Ponndorf-Verley reduction reaction to obtain TM9, TM10, TM11 and TM12. The reaction catalyst is aluminum isopropoxide or aluminum tert-butoxide, the reaction solvent is chloroform or toluene, the reducing agent used in the reaction is isopropanol or cyclohexanol, the reaction temperature ranges from 60 to 100 °C, and the reaction time is 1-8 h. The ratio of the reaction products TM9, TM10, TM11 and TM12 is related to the reaction time. A small amount of by-products with the carbonyl group at the 20-position being reduced will be generated. TM10 and TM11 are the main reaction products corresponding to TM2 and TM1, respectively. The four products TM12, TM9, TM11 and TM10 can be successively obtained by column chromatography separation.

Synthesis of TM25 - TM28

On the basis of preparing TM9, TM10, TM11 and TM12, a bromination reaction is carried out first, followed by a substitution reaction to obtain the compounds TM25 - TM28 of the present invention, namely TM25, TM26, TM27 and TM28.

Specifically, on the basis of preparing TM9, TM10, TM11 and TM12, the corresponding 22-methyl group is subjected to a bromination reaction first, and then a substitution reaction with 4-cyanopyrazole is carried out to generate the target products. The bromination reagent used in the bromination reaction is bromine, the catalyst is hydrobromic acid, the solvent is methanol or ethanol, the reaction time ranges from 0.5 to 2 h, the temperature ranges from 20 °C to 40 °C, and the reaction can be quenched by adding an aqueous solution of sodium bicarbonate, followed by extraction, liquid separation and drying. The product can be directly used in the next step without purification. The base used in the substitution reaction is potassium carbonate, the solvent is acetone or tetrahydrofuran, the reaction temperature ranges from 25 °C to 40 °C, and the reaction time ranges from 0.5 to 3 h. The product TM25 - TM28, namely TM25, TM26, TM27 and TM28, can be obtained by adding water and dichloromethane for extraction, liquid separation, drying and column chromatography.

Route L2

Using dydrogesterone as a starting material, TM1 and TM2 are first prepared through the following process route, and then TM13 - TM16 are prepared using TM1 and TM2, and then TM17 - TM20 are prepared from TM13 - TM16.

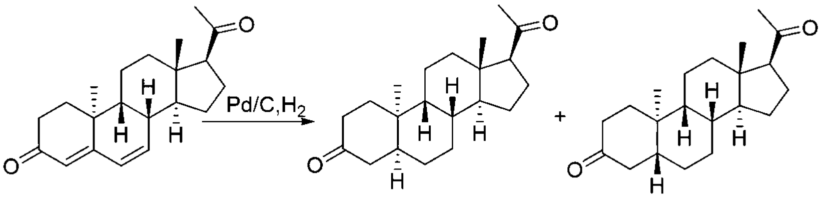


Dydrogesterone TM1 TM2

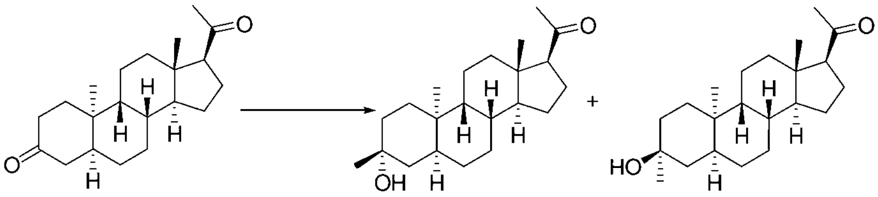

TM1 TM13 TM14

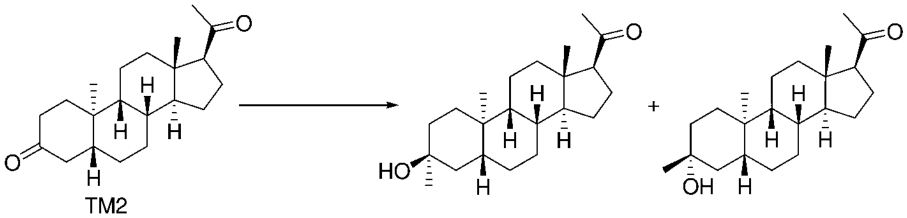

TM2 TM15 TM16

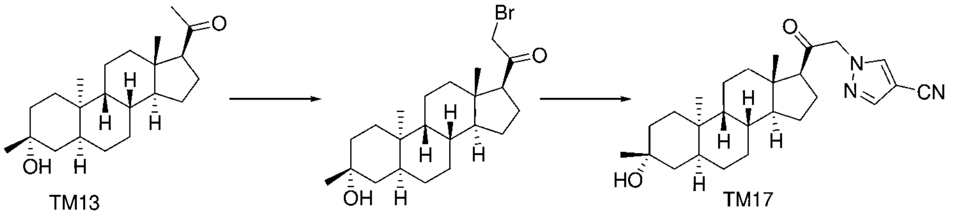

TM13 TM17

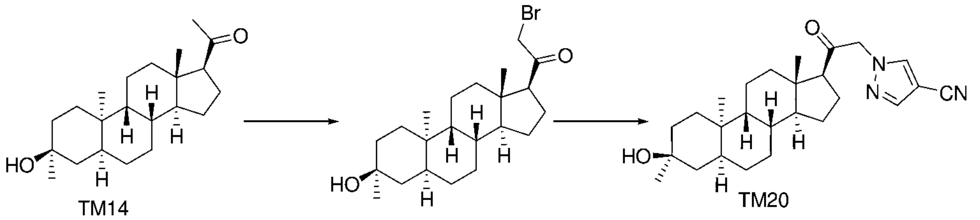

TM14 TM20

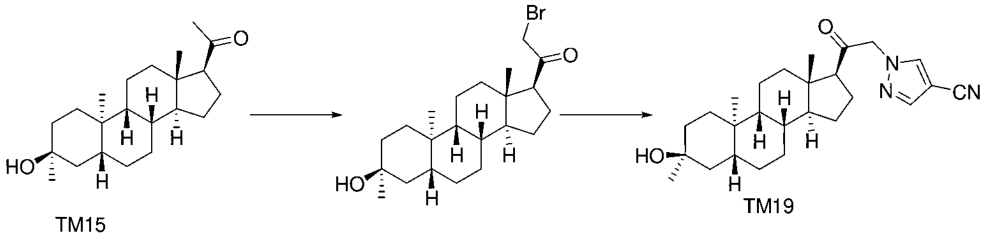

TM15 TM19

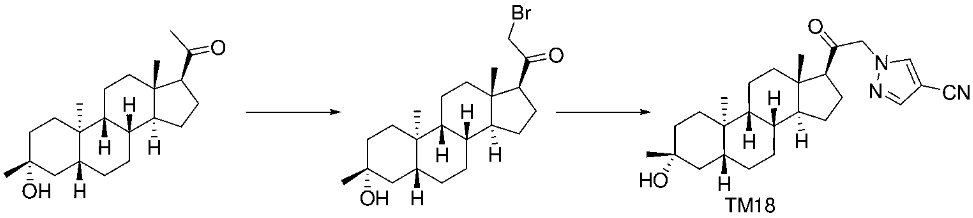

TM16 TM18

The synthesis of TM1 and TM2 is the same as that in Route L1.

Synthesis of TM13 - TM16

On the basis of preparing TM1 and TM2, TM13, TM14, TM15 and TM16 can be obtained by selectively adding to the carbonyl group at the 3-position of TM1 or TM2. The reagents used in the addition reaction are methylmagnesium chloride or methylmagnesium bromide, the solvents used are toluene or tetrahydrofuran, the reaction temperature ranges from -70 °C to -30 °C, the reaction time ranges from 15 minutes to 1 hour, and the amount of the Grignard reagent added is 1.2 to 2 times the amount of moles of the starting material. The reaction generates the target products and by-products of double addition at the 3,20-positions. TM13 and TM14, as well as TM15 and TM16 can be obtained by column chromatography respectively.

Synthesis of TM17 - TM20

On the basis of preparing TM13, TM14, TM15 and TM16, a bromination reaction is carried out first, followed by a substitution reaction to obtain the compounds TM17 - TM20 of the present invention, namely TM17, TM18, TM19 and TM20.

Specifically, on the basis of preparing TM13, TM14, TM15 and TM16, the corresponding 22-methyl group is subjected to a bromination reaction first, and then a substitution reaction with 4-cyanopyrazole is carried out to generate the target products. The bromination reagent used in the bromination reaction is bromine, the catalyst is hydrobromic acid, the solvent is methanol or ethanol, the reaction time ranges from 0.5 to 2 h, the temperature ranges from 20 °C to 40 °C, and the reaction can be quenched by adding an aqueous solution of sodium bicarbonate, followed by extraction, liquid separation and drying. The product can be directly used in the next step without purification. The base used in the substitution reaction is potassium carbonate, the solvent is acetone or tetrahydrofuran, the reaction temperature ranges from 25 °C to 40 °C, and the reaction time ranges from 0.5 to 3 h. The product TM17 - TM20, namely TM17, TM18, TM19 and TM20, can be obtained by adding water and dichloromethane for extraction, liquid separation, drying and column chromatography.

During the preparation process of the compounds of the present invention, the protection or deprotection reactions of functional groups are carried out according to known methods, such as the methods described in the following literature: Protective Groups in Organic Synthesis, 4th Edition (Theodora W. Greene, Peter G. M. Wuts), Wiley - Interscience (2007).

Examples of alcohol hydroxyl protecting groups include: ether-type protecting groups such as methoxymethyl ether (-OMOM), trimethylsilyl ether (-OTMS), tetrahydropyranyl ether, etc.; carboxylic ester-type protecting groups such as acetate (-OAc), etc.; sulfonate-type protecting groups such as p-toluenesulfonate (-OTs), etc.

Examples of ketone carbonyl protecting groups include: ketal-type protecting groups such as dimethyl ketal, etc.; cyclic ketal-type protecting groups such as 1,3-dioxolane, 1,3-dioxane, etc.; oxime-type protecting groups such as O-methyl oxime, etc.; hydrazone-type protecting groups such as N,N-dimethyl hydrazone, etc.

The following specific examples will further illustrate the present invention.

**Example 1:** Synthesis of TM9 - TM12

Weigh 10.0 g of dydrogesterone, add 100 mL of methanol, 100 mL of THF, and 1.0 g of wet palladium on carbon (with a content of 5%), and let the reaction proceed overnight at room temperature. TLC (P:E = 2:1) shows that the fluorescence disappears, indicating the completion of the reaction. Filter to remove the palladium on carbon, add 10 mL of water, then add 5 mL of hydrochloric acid, stir. TLC shows that the 3-position ketal has been completely decomposed. Evaporate the solvent under reduced pressure, add 50 mL of isopropyl ether for slurrying, and obtain 4.5 g of TM1. Evaporate the mother liquor under reduced pressure and perform column chromatography (PE:EA = 8:1) to obtain 0.8 g of TM2 and most of the mixture of TM1 and TM2.

Weigh 5.0 g of the hydrogenation product of dydrogesterone synthesized above, which is the mixture of TM1 and TM2, add 150 mL of chloroform to dissolve it, add 5.0 g of aluminum isopropoxide and 7.5 mL of cyclohexanol, stir and reflux for 6 h. TLC (P:E = 2:1) shows that most of the starting materials have been converted and multiple product spots with increased polarity are generated. Perform column chromatography to obtain 0.15 g of TM9, 0.78 g of TM10, 2.55 g of TM11 and 0.10 g of TM12.

After detection, the results are as follows:

TM9: $^1$H NMR (400 MHz, $CDCl_3$) δ 4.07 (d, *J* = 3.2 Hz, 1H), 2.55 (t, *J* = 9.2 Hz, 1H), 2.15 (m, 1H), 2.11 (s, 3H), 1.95-1.84 (m, 2H), 1.84-1.72 (m, 6H), 1.72-1.57 (m, 6H), 1.55-1.45 (m, 2H), 1.45-1.31 (m, 3H), 1.30-1.21 (m, 1H), 1.20-1.15 (m, 1H), 1.12 (s, 3H), 0.65 (s, 3H).
$^{13}$C NMR (100 MHz, $CDCl_3$) δ 209.62, 77.24, 66.92, 65.02, 61.92, 49.73, 43.97, 37.89, 36.80, 35.78, 34.34, 34.16, 31.74, 31.36, 29.06, 25.56, 23.68, 22.31, 21.23.

TM10: $^1$H NMR (400 MHz, $CDCl_3$) δ 3.61 (ddd, *J* = 16.1, 10.8, 5.0 Hz, 1H), 2.57 (s, 1H), 2.11 (s, 3H), 1.92-1.83 (m, 2H), 1.74-1.82 (s, 4H), 1.74-1.66 (m, 3H), 1.65-1.61 (m, 1H), 1.54 -1.45 (m, 2H), 1.45-1.40 (m, 1H), 1.37-1.30 (m, 3H), 1.30-1.27 (m, 1H), 1.15-1.08 (m, 3H), 1.01 (s, 3H), 0.97-0.90 (m, 1H), 0.87-0.81 (m, 1H), 0.63 (s, 3H).
$^{13}$C NMR (100 MHz, $CDCl_3$) δ 209.65, 71.32, 64.80, 48.68, 45.33, 44.81, 44.33, 38.85, 37.68, 37.60, 37.42, 35.49, 31.46, 30.96, 29.70, 29.19, 25.40, 25.27, 22.19, 21.93, 16.43, 12.85.
TM11: $^1$H NMR (400 MHz, $CDCl_3$) δ 3.69-3.57 (m, 1H), 2.56 (t, *J* = 9.2 Hz, 1H), 2.18 -2.12 (m, 1H), 2.11 (s, 4H), 2.03 (t, *J* = 5.2 Hz, 1H), 1.95 (dt, *J* = 14.6, 3.5 Hz, 1H), 1.92-1.83 (m, 2H), 1.83-1.74 (m, 4H), 1.73-1.65 (m, 4H), 1.63-1.58 (m, 2H), 1.54 -1.45 (m, 1H), 1.44-1.24 (m, 4H), 1.20-1.12 (m, 1H), 1.10 (s, 3H), 0.91 (td, *J* = 14.2, 3.7 Hz, 1H), 0.63 (s, 3H).
$^{13}$C NMR (100 MHz, $CDCl_3$) δ 209.68, 71.87, 64.87, 48.72, 44.32, 42.18, 37.72, 37.21, 36.57, 35.62, 34.56, 31.47, 31.38, 31.12, 29.69, 25.45, 23.33, 22.18, 22.16, 21.58, 12.65.
TM12: $^1$H NMR (400 MHz, $CDCl_3$) δ 4.03 (t, *J* = 2.4 Hz 1H), 2.56 (t, *J* = 9.2 Hz, 1H), 2.18 - 2.07 (m, 1H), 2.11 (s, 3H), 1.87 - 1.75 (m, 4H), 1.75 - 1.63 (m, 5H), 1.63 - 1.58 (m, 3H), 1.53 (s, 1H), 1.52 - 1.42 (m, 3H), 1.42-1.35 (m, 1H), 1.25 - 1.19 (m, 1H), 1.11 (dd, *J* = 11.6, 6.2 Hz, 1H), 1.07 - 1.00 (m, 1H), 0.98 (s, 3H), 0.63 (s, 3H).
$^{13}$C NMR (100 MHz, $CDCl_3$) δ 209.67, 66.52, 64.85, 48.76, 45.28, 44.39, 39.24, 38.05, 37.65, 35.65, 35.50, 34.12, 31.46, 29.06, 28.70, 25.41, 24.91, 22.19, 21.46, 15.35, 12.81.

**Example 2:** Synthesis of TM26, TM27, TM25 and TM28

Synthesis of TM26

Weigh 0.36 g of TM10, add 15 mL of methanol to dissolve it, add 3 drops of hydrobromic acid (48%), 0.5 g of bromine, and stir the reaction at 28 °C for 1 h. TLC shows that the conversion is complete. Add an aqueous solution of sodium bicarbonate to quench the reaction, then add 30 mL of DCM for extraction, separate the layers, dry, and evaporate the solvent under reduced pressure to obtain the brominated product. Add 20 mL of acetone, 0.4 g of potassium carbonate, and 0.2 g of 4-cyanopyrazole to the brominated product, and react at 28 °C for 1 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.16 g of the product TM26.

After detection, the results are as follows:

TM26: $^1$H NMR (400 MHz, CDCl3) δ 7.86 (s, 1H), 7.80 (s, 1H), 4.95 (dd, J = 43.2, 17.9 Hz, 2H), 3.71 - 3.51 (m, 1H), 2.65 (t, J = 8.9 Hz, 1H), 2.17 (m, 1H), 1.96 - 1.63 (m, 13H), 1.62 - 1.43 (m, 4H), 1.42 - 1.34 (m, 2H), 1.34 - 1.28 (m, 2H), 1.21 - 1.08 (m, 3H), 1.02 (s, 3H), 0.98 - 0.76 (m, 4H), 0.68 (s, 3H).
$^{13}$C NMR (100 MHz, CDCl3) δ 202.02, 142.35, 136.08, 113.24, 93.10, 71.20, 61.94, 61.58, 48.88, 45.39, 45.13, 44.74, 38.80, 37.65, 37.60, 37.42, 35.45, 30.90, 29.15, 25.43, 25.20, 22.37, 21.87, 16.50, 13.25.

Synthesis of TM27

Weigh 1.21 g of TM11, add 30 mL of methanol to dissolve it, add 10 drops of hydrobromic acid (48%), 1.80 g of bromine, and stir the reaction at 25 °C for 1.5 h. TLC shows that the conversion is complete. Add an aqueous solution of sodium bicarbonate to quench the reaction, then add 60 mL of DCM for extraction, separate the layers, dry, and evaporate the solvent under reduced pressure to obtain the brominated product. Add 40 mL of acetone, 1.8 g of potassium carbonate, and 0.6 g of 4-cyanopyrazole to the brominated product, and react at 28 °C for 1 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.83 g of the product TM27.

Synthesis of TM25 and TM28

TM25 and TM28 were synthesized by adopting the similar process steps as those for the synthesis of TM26 and TM27 above.

**Example 3:** Synthesis of TM13, TM14, TM15 and TM16

Synthesis of TM13 and TM14

Weigh 3.16 g of TM1, add 63 mL of toluene to dissolve it, cool the temperature down to -40 °C, add dropwise 9.3 mL of a tetrahydrofuran solution of methylmagnesium chloride (1.6 M). After the addition is completed, continue the

reaction for 20 minutes. TLC (PE:DCM:EA = 4:2:1) shows that most of the starting material has reacted and four product spots are generated. Add methanol to quench the reaction, add ethyl acetate and an aqueous solution of ammonium chloride, perform extraction, separate the layers, dry, and perform column chromatography to obtain 0.52 g of TM13 and 0.25 g of TM14.

After detection, the results are as follows.

TM13: $^1$H NMR (400 MHz, CDCl3) δ 2.57 (t, J = 9.2 Hz, 1H), 2.18-2.12 (m, 1H), 2.10 (s, 3H), 1.95-1.83 (m, 3H), 1.82-1.70 (m, 6H), 1.70-1.58 (m, 4H), 1.46-1.34 (m, 3H), 1.34-1.28 (m, 1H), 1.28-1.25 (m, 1H), 1.22 (s, 3H), 1.19 (m, 1H), 1.15 (s, 3H), 1.14-1.04 (m, 2H), 0.62 (s, 3H).
$^{13}$C NMR (100 MHz, CDCl3) δ 209.64, 70.11, 64.84, 48.68, 44.36, 39.41, 38.27, 37.72, 35.53, 34.47, 34.33, 33.72, 31.67, 31.48, 30.29, 29.93, 25.49, 22.80, 22.15, 21.98, 21.64, 12.59.
TM14: $^1$H NMR (400 MHz, CDCl3) δ 2.56 (t, J = 9.2 Hz, 1H), 2.10 (s, 3H), 2.04 (t, J = 5.9 Hz, 1H), 1.94 - 1.85 (m, 3H), 1.85 - 1.69 (m, 7H), 1.68 - 1.54 (m, 4H), 1.51 (dd, J = 13.6, 3.3 Hz, 1H), 1.45 - 1.34 (m, 3H), 1.25 (s, 3H), 1.20 (m, 1H), 1.12 (s, 3H), 1.04 - 0.94 (m, 1H), 0.63 (s, 3H).
$^{13}$C NMR (100 MHz, CDCl3) δ 209.63, 71.97, 64.87, 48.74, 44.31, 41.25, 41.21, 37.74, 36.47, 36.02, 35.95, 34.46, 31.45, 30.80, 29.84, 26.06, 25.44, 23.22, 22.15, 22.06, 21.55, 12.68.

Synthesis of TM15 and TM16

Weigh 1.0 g of TM2, add 30 mL of toluene to dissolve it, cool the temperature down to -60 °C, add dropwise 2.5 mL of a tetrahydrofuran solution of methylmagnesium chloride (1.6 M). After the addition is completed, continue the reaction for 30 minutes. TLC (PE:DCM:EA = 4:2:1) shows that most of the starting material has reacted and four product spots are generated. Add methanol to quench the reaction, add ethyl acetate and an aqueous solution of ammonium chloride, perform extraction, separate the layers, dry, and perform column chromatography to obtain 0.18 g of TM15 and 0.53 g of TM16.

After detection, the results are as follows.

TM16: $^1$H NMR (400 MHz, $CDCl_3$) δ 2.57 (t, *J* = 9.2 Hz, 1H), 2.18 - 2.07 (m, 1H), 2.11 (s, 3H), 1.90 - 1.74 (m, 5H), 1.74-1.65 (m, 4H), 1.65 - 1.59 (m, 2H), 1.54-1.47 (m, 3H), 1.38 (t, *J* = 5.4 Hz, 1H), 1.34 - 1.27 (m, 2H), 1.26 (s, 4H), 1.18 - 1.05 (m, 3H), 1.01 (s, 3H), 0.63 (s, 3H).
$^{13}$C NMR (100 MHz, $CDCl_3$) δ 209.67, 71.46, 64.79, 48.76, 45.45, 44.33, 44.20, 42.92, 38.38, 37.97, 37.58, 35.96, 35.50, 31.47, 29.14, 27.05, 25.44, 25.25, 22.20, 21.86, 15.94, 12.87.

**Example 4:** Synthesis of TM17, TM18, TM19, and TM20

Synthesis of TM17

Weigh 0.66 g of TM13, add 20 mL of methanol to dissolve it, add 6 drops of hydrobromic acid (48%), 0.96 g of bromine, and stir the reaction at 25 °C for 2 h. TLC (PE:DCM:EA = 4:2:1) shows that the conversion is complete. Add an aqueous solution of sodium bicarbonate to quench the reaction, then add 50 mL of DCM for extraction, separate the layers, dry, and evaporate the solvent under reduced pressure to obtain the brominated product. Add 30 mL of acetone, 1.2 g of potassium carbonate, and 0.5 g of 4-cyanopyrazole to the brominated product, and react at 28 °C for 1.5 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.37 g of the product TM17.

After detection, the results are as follows.

TM17: $^1$H NMR (400 MHz, $CDCl_3$) δ 7.85 (s, 1H), 7.80 (s, 1H), 4.95 (dd, *J* = 45.5, 17.9 Hz, 2H), 2.65 (t, *J* = 9.0 Hz, 1H), 2.24-2.13(m, 1H), 2.03-1.90 (m, 2H), 1.89 - 1.59 (m, 11H), 1.50 - 1.36 (m, 3H), 1.35 - 1.28 (m, 2H), 1.23 (s, 3H), 1.21-1.17 (m, 2H), 1.15 (s, 3H), 0.68 (s, 3H).
$^{13}$C NMR (100 MHz, $CDCl_3$) δ 201.97, 142.36, 136.02, 113.21, 93.16, 70.13, 62.01, 61.62, 48.91, 45.45, 39.38, 38.23, 37.82, 35.56, 34.48, 34.34, 33.70, 31.70, 30.22, 29.95, 25.53, 22.75, 22.35, 21.98, 21.61, 13.01.

Synthesis of TM20

Weigh 0.36 g of TM14, add 15 mL of methanol to dissolve it, add 3 drops of hydrobromic acid (48%), 0.50 g of bromine, and stir the reaction at 25 °C for 2 h. TLC (PE:DCM:EA = 4:2:1) shows that the conversion is complete. Add an aqueous solution of sodium bicarbonate to quench the reaction, then add 50 mL of DCM for extraction, separate the layers,

dry, and evaporate the solvent under reduced pressure to obtain the brominated product. Add 20 mL of acetone, 0.5 g of potassium carbonate, and 0.2 g of 4-cyanopyrazole to the brominated product, and react at 28 °C for 1.5 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.15 g of the product TM20.

After detection, the results are as follows.

TM20: $^{1}$H NMR (400 MHz, $CDCl_3$) δ 7.86 (s, 1H), 7.80 (s, 1H), 4.94 (dd, *J* = 42.0, 17.8 Hz, 2H), 2.64 (t, *J* = 9.0 Hz, 1H), 2.25 - 2.10 (m, 1H), 2.07 (t, *J* = 6.0 Hz, 1H), 1.98 - 1.65 (m, 11H), 1.51 (dd, *J* = 13.6, 3.5 Hz, 1H), 1.46 - 1.34 (m, 4H), 1.25 (s, 4H), 1.23-1.15 (m, 2H), 1.13 (s, 3H), 1.01 (td, *J* = 14.3, 4.0 Hz, 1H), 0.69 (s, 3H).
$^{13}$C NMR (100 MHz, $CDCl_3$) δ 201.94, 142.36, 136.03, 113.21, 93.15, 71.97, 62.05, 61.59, 48.97, 45.38, 41.19, 41.17, 37.83, 36.44, 36.01, 35.96, 34.45, 30.75, 29.85, 26.12, 25.48, 23.18, 22.35, 22.07, 21.52, 13.11.

Synthesis of TM19

Weigh 0.19 g of TM15, add 10 mL of methanol to dissolve it, add 2 drops of hydrobromic acid (48%), 0.35 g of bromine, and stir the reaction at 25 °C for 2 h. TLC (PE:DCM:EA = 4:2:1) shows that the conversion is complete. Add an aqueous solution of sodium bicarbonate to quench the reaction, then add 30 mL of DCM for extraction, separate the layers, dry, and evaporate the solvent under reduced pressure to obtain the brominated product. Add 20 mL of acetone, 0.3 g of potassium carbonate, and 0.15 g of 4-cyanopyrazole to the brominated product, and react at 28 °C for 2 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.05 g of the product TM19.

After detection, the results are as follows.

TM19: $^{1}$H NMR (400 MHz, $CDCl_3$) δ 7.83 (d, *J* = 20.7 Hz, 2H), 4.95 (dd, *J* = 42.4, 17.9 Hz, 2H), 2.64 (t, *J* = 9.0 Hz, 1H), 2.17 (ddd, *J* = 13.6, 11.6, 5.8 Hz, 1H), 1.96 - 1.78 (m, 5H), 1.78 - 1.61 (m, 6H), 1.56 - 1.45 (m, 3H), 1.41 (t, *J* = 13.2 Hz, 2H), 1.30-1.22 (m, 4H), 1.21 (s, 4H), 1.11 - 1.02 (m, 1H), 0.94 (s, 3H), 0.69 (s, 3H).
$^{13}$C NMR (100 MHz, $CDCl_3$) δ 202.00, 142.36, 136.03, 113.21, 93.15, 69.80, 62.01, 61.59, 48.99, 45.44, 45.00, 41.53, 41.06, 37.73, 37.55, 35.88, 35.50, 34.56, 31.66, 29.02, 25.45, 24.81, 22.37, 21.62, 15.41, 13.25.

Synthesis of TM18

Weigh 0.51 g of TM16, add 20 mL of methanol to dissolve it, add 5 drops of hydrobromic acid (48%), 0.74 g of bromine, and stir the reaction at 28 °C for 1 h. TLC (PE:DCM:EA = 4:2:1) shows that the conversion is complete. Add an aqueous solution of sodium bicarbonate to quench the reaction, then add 50 mL of DCM for extraction, separate the layers, dry, and evaporate the solvent under reduced pressure to obtain the brominated product. Add 30 mL of acetone, 1.2 g of potassium carbonate, and 0.3 g of 4-cyanopyrazole to the brominated product, and react at 32 °C for 1 h. TLC (PE:EA = 1:1) shows that a product with increased polarity is generated. Add water and dichloromethane for extraction, separate the layers, dry, perform column chromatography to obtain the product, and then use water and isopropyl ether for slurrying to obtain 0.18 g of the product TM18.

After detection, the results are as follows:

TM18: $^{1}$H NMR (400 MHz, $CDCl_3$) δ 7.86 (s, 1H), 7.80 (s, 1H), 4.95 (dd, *J* = 44.8, 17.9 Hz, 2H), 2.65 (t, *J* = 9.0 Hz, 1H), 2.23 - 2.07 (m, 1H), 1.95 - 1.73 (m, 7H), 1.73 - 1.65 (m, 3H), 1.64-1.57 (m, 2H), 1.54 - 1.48 (m, 2H), 1.41 (dd, *J* = 6.9, 5.1 Hz, 1H), 1.35 - 1.28 (m, 2H), 1.26 (s, 4H), 1.15 - 1.08 (m, 2H), 1.02 (s, 3H), 0.69 (s, 3H).
$^{13}$C NMR (100 MHz, $CDCl_3$) δ 201.99, 142.36, 136.04, 113.22, 93.14, 71.39, 61.94, 61.58, 48.96, 45.39, 45.26, 44.13, 42.84, 38.34, 37.98, 37.65, 35.91, 35.47, 29.10, 27.06, 25.47, 25.17, 22.39, 21.81, 16.01, 13.28.

## Example 5

### I. Molecular Docking Simulation of Compounds with GABAA Receptor and Hormone Receptor

The docking scores of the following compounds were calculated using the docking software MOE (Molecular Operating Environment, an integrated software system for pharmaceuticals and life sciences developed by Chemical Computing Group ULC in Canada). The scoring of the MOE software is based on the results calculated from parameters such as electrostatic parameters, hydrogen bond binding, molecular attraction, and molecular orbitals between molecules. The more negative the score is, the lower the free energy is, the more stable the conformation is, and the better the

binding is.

The following compound (the difference between it and TM17 is that the methyl group at the C10 position is in the unflipped β configuration) and TM17 were docked and scored with the GABAA receptor. The docking scores were -5.39 and -5.72 respectively, indicating that the 10α configuration compound TM17 obtained after the flip binds better to the GABAA receptor and is expected to have higher activity.

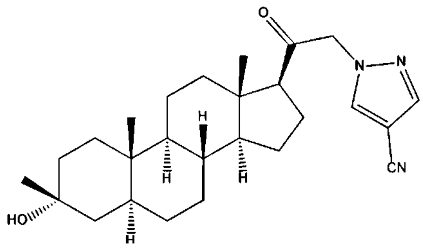

In addition, TM17 was docked and scored with the androgen receptor and the estrogen receptor respectively. The binding score of TM17 to the androgen receptor was -5.30, and the binding score of TM17 to the estrogen receptor was -4.97, both of which were lower than the binding score of -5.72 to the GABAA receptor. This indicates that the compound TM17 has more selectivity in binding to the GABAA receptor, thereby reducing side effects on non-target receptors.

**II. Pharmacological Experiments: Effects of the Compounds in the Examples of the Present Invention on Chloride Channels and Acute Depression in Mice**

**1. Purpose of the Experiment**

The chloride channel model was constructed using human glioma cells, and the acute depression model was prepared using mice to comprehensively evaluate the effects of neurosteroid compounds on chloride channels and the mouse model of acute depression.

**2. Experimental Materials**

**Samples:** TM17, TM18, TM19, TM20; **Control sample:** Solvent control - edible oil.

**Experimental cells:** Human glioma cells U251, provided by the Cancer Research Institute of Central South University, and the culture conditions were high-glucose DMEM + 10% FBS.

**Experimental animals:** 60 ICR mice, with an equal number of males and females. The weight range of female mice was 14.55 - 19.22 g, and the weight range of male mice was 14.84 - 17.44 g, provided by Hunan Silaike Jingda Laboratory Animal Co., Ltd.

**Rearing environment:** Environmental grade: Ordinary grade; Temperature: 22 - 26 °C; Relative humidity: 40% - 70%; Cage: 465 × 300 × 200 $mm^3$; Feed and drinking water: Complete pellet SPF mouse feed, 10 kg/bag, barreled purified water; Bedding: Corncob bedding.

**3. Experimental Methods**

**3.1. Cell Experimental Methods**

MQAE is a chloride ion fluorescent probe, with bromide ion as the paired anion. Its maximum excitation wavelength is approximately 355 nm, and its maximum emission wavelength is approximately 460 nm. When the intracellular chloride ion concentration increases, the fluorescence intensity of MQAE decreases proportionally with the increase in the chloride ion concentration, that is, the fluorescence intensity is inversely proportional to the chloride ion concentration. MQAE is prepared with Krebs-HEPES buffer containing a high concentration of chloride ions. When U251 cells are incubated with MQAE, the intracellular chloride ion concentration of U251 cells can be increased. Samples are prepared with chlorine-free buffer, and after mixing, a chloride ion concentration difference is generated between the inside and outside of the cells. The GABAa receptor is activated by using γ-aminobutyric acid (GABA) to open the chloride ion channel, allowing the intracellular chloride ions to flow out and increasing the fluorescence intensity of the cells. Through the fluorescence intensity, the opening degree of the chloride ion channel can be reflected, and the influence of neurosteroid compounds on the chloride ion channel can be detected.

**Reagent Preparation**

Chloride-free Buffer: 20 mM HEPES, 2.4 mM $K_2HPO_4$, 1 mM $KH_2PO_4$, 1 mM $CaSO_4$, 130 mM $NaNO_3$, 10 mM Glucose;
Chloride-containing Buffer: 20 mM HEPES, 2.4 mM $K_2HPO_4$, 1 mM $KH_2PO_4$, 1 mM $CaSO_4$, 130 mM NaCl, 10 mM Glucose;
Krebs-HEPES Buffer: 20 mM HEPES, 128 mM NaCl, 2.5 mM KCl, 2.7 mM $CaCl_2$, 1 mM $MgCl_2$, 16 mM glucose;
Sample Preparation Matrix (containing 5 μM GABA): Take 100 mL of the chloride-free buffer and mix it evenly with 100 mL of serum-free DMEM at a ratio of 1:1;
Preparation of MQAE Dye: Take 1 vial (20 mg) of MQAE dye. The relative molecular weight of MQAE is 326.19. Add 61.31 μL of Krebs-HEPES buffer to prepare a 1 M solution. Take 30 μL and use Krebs-HEPES buffer to prepare 6 mL, thus obtaining the MQAE dye working solution with a final concentration of 5 mM.

**Preparation of Working Solutions of Samples to be Tested**

TM17, TM18, TM19, and TM20 were prepared into working concentrations of 20 μM using the sample preparation matrix.

**Cell Preparation and Setting of Activity Detection Groups**

U251 cells were collected after routine digestion and then inoculated into 96-well plates at an inoculation density of $1 \times 10^5$ cells per well. They were placed in an incubator and cultured overnight. After the cells adhered to the wall, the cells were washed 3 times with the chloride-free buffer. Then, 100 μL per well of the MQAE working solution was added to incubate the cells under the incubation conditions of avoiding light, at 37 °C for 30 minutes.

TM17 - TM20 were the samples to be tested. All the samples to be tested were prepared into working solutions with a final concentration of 20 μM using the chloride-free buffer. The chloride-free buffer without samples was set as the blank control. The group settings are shown in Table 1 below.

Table 1: Samples used for testing

| sample | concentration (μM) | MQAE incubation time | excitation light color | Emitted light color | Number of complex holes (pcs) |
|---|---|---|---|---|---|
| blank control | -- | 30min | blue | green | 6 |
| TM17 | 10 | 30min | blue | green | 6 |
| TM18 | 10 | 30min | blue | green | 6 |
| TM19 | 10 | 30min | blue | green | 6 |
| TM20 | 10 | 30min | blue | green | 6 |

**Detection**

Turn on the fluorescence microscope, turn on the fluorescence light source, turn off the visible light source, adjust the B/G intensity of the fluorescence light source to 85 and the UV intensity to 0. After the brightness of the emitted light becomes stable (about 15 minutes), take the 96-well culture plate after the incubation in the dark, and add the prepared working solutions containing samples into the 96-well plate at a volume of 100 μL per well. One minute later, observe under the fluorescence microscope and take pictures of the fields of view where the cell growth is relatively uniform under a 10× objective lens. Take 6 pictures for each sample, and use the Image J software to conduct semi-quantitative analysis on the average fluorescence intensity of the photographed fields of view. The results are expressed as mean ± standard deviation.

**3.2. Animal Test Methods**

**Grouping and Identification of Animals**

The experimental animals were administered drugs according to the grouping after 3 days of adaptive feeding.

The experimental groups included the model group, the compound TM1 group, and the compound TM2 group. For each group of compounds to be tested, edible oil was used as the vehicle, and the administration route for all animals was intraperitoneal injection. The vehicle control group was given the same volume of edible oil by intraperitoneal injection, and the administration volume for each group was 20 mL/kg.

The details of the test doses and groupings are shown in Table 2.

**Setting of the Administration Cycle**

Brexanolone is used to treat postpartum depression. Its usage and dosage are continuous intravenous injection for 60 hours, with a dosage of 100 mg every 12 hours. At the beginning of the administration, it is necessary to gradually increase the drug use rate, and at the end of the administration, it is necessary to gradually decrease the drug use rate. Therefore, the total drug use within the treatment cycle is 450 mg. Referring to the Brexanolone administration cycle, the administration cycle was set to 3 days, with one administration in the morning every day.

The clinical dosage of Brexanolone is approximately 200 mg/d. Calculated based on an adult weight of 70 kg, the clinical dose of Brexanolone is 2.857 mg/kg/d. The equivalent dose conversion coefficient between humans and mice calculated according to body surface area is 0.0026. Taking the weight of mice as 20 g, the converted dose for mice is: 2.857 mg/kg/d $\times$ 70 kg $\times$ 0.0026 / 0.02 kg = 25.999 mg/kg/d, which is counted as 26.0 mg/kg/d.

This study is for validity verification, and single-dose detection is used for the time being. The administration cycle and clinical dosage of the compounds of the present invention refer to the administration cycle and clinical dosage of Brexanolone mentioned above.

Table 2 Design of Test Doses and Groupings

| group | dosage (mg/kg) | concentration (mg/mL) | administration volume | animal number |
|---|---|---|---|---|
| model control group | -- | -- | 20mL/kg | 1F01-1F05, 1M01-1M05 |
| TM17 | 26.0 | 1.3 | 20mL/kg | 3F01-3F05, 3M01-3M05 |
| TM18 | 26.0 | 1.3 | 20mL/kg | 4F01-4F05, 4M01-4M05 |
| TM19 | 26.0 | 1.3 | 20mL/kg | 5F01-5F05, 5M01-5M05 |
| TM20 | 26.0 | 1.3 | 20mL/kg | 6F01-6F05, 6M01-6M05 |

**Detection Indicators and Detection Methods**

The detection indicator is the behavioral changes of mice in the last 4 minutes within 6 minutes, including the time when the immobile state first appears and the duration of the immobile state. Among them, the immobile state refers to the state in which the animal gives up active struggling and remains completely still.

Detection method: 24 hours after the administration of drugs to animals in each group, the animals in each group were placed in the laboratory for 30 minutes to 1 hour to reduce the animals' anxiety about the new environment. Install a video camera in front of the tail suspension box, set the corresponding parameters in the software, and enter the information such as the animal number and status. Take out the experimental animals, mark them with a marker pen at a position 3 mm away from the tip of the tail, fix the tail at the marked position with medical tape. After the tails of the animals in the same group are fixed with tape, quickly suspend the mice in the same group into the tail suspension box device, turn on the behavioral software to record the behavioral situation for 6 minutes, and analyze the behavioral changes in the last 4 minutes.

After the experiment, remove the tape from the tails of the mice and put the animals back into the breeding cages.

**4. Experimental Results**

**4.1. Influence of TM Series Samples on Chloride Channels of U251 Cells**

GABA activates the GABAa receptor on the neuronal cell membrane to open the chloride channel, resulting in the influx of chloride ions into the cell, generating heterogeneous postsynaptic potentials to play a role and inhibiting neuronal excitation. In this experiment, U251 cells were incubated with the MQAE probe prepared with the Krebs-HEPES buffer containing a high concentration of chloride ions. After the chloride ion concentration inside and outside the cell reached equilibrium, a cell model with high intracellular chloride ions could be constructed. Then, GABA was used to activate the chloride ion GABAa receptor to open the chloride ion channel.

The TM series compounds are neurosteroid compounds with GABAa receptor positive allosteric agent activity,

which can prolong the time for GABA to activate the GABAa receptor and prolong the opening time of the chloride ion channel. After adding the TM series samples prepared with the chlorine-free matrix outside the U251 cells, the chloride ion concentration outside the U251 cells decreased. The TM series samples can prolong the opening time of the chloride ion channel, and chloride ions continuously flow out of the cells. The fluorescence display intensity of MQAE is inversely related to the intracellular chloride ion concentration. Therefore, the intracellular fluorescence intensity reflects the duration of the opening of the chloride ion channel and indirectly reflects the activity of the TM series samples.

The average fluorescence intensity of the vehicle control group was 42.543 ± 4.202. The average fluorescence intensities of TM17, TM18, TM19, and TM20 were 47.499 ± 2.240, 48.621 ± 1.006, 51.201 ± 3.371, and 47.562 ± 2.659 respectively. Compared with the vehicle control group, the average fluorescence intensities of each sample group were significantly increased, and there were significant differences ($P < 0.05$), indicating that the TM series samples can significantly prolong the opening time of the chloride ion channel activated by GABA.

For the detailed analysis of the average fluorescence intensity after the TM series samples intervened in U251 cells, see Table 3.

Table 3 Fluorescence Result Analysis result (n = 6)

| group | Average fluorescence intensity (mean ± standard deviation) | *P* - value |
|---|---|---|
| vehicle control group | 42.543±4.202 | -- |
| TM17 | 47.499±2.240 | 0.004** |
| TM18 | 48.621±1.006 | 0.001** |
| TM19 | 51.201±3.371 | 0.000** |
| TM20 | 47.562±2.659 | 0.003** |
| Note: "*" indicates that compared with the vehicle control group, P < 0.05; "**" indicates that compared with the vehicle control group, P < 0.01. | | |

### 4.2. The Effect of TM Series Samples on the Mouse Model of Acute Depression

Mice were placed in an inverted suspension position under the head. In the initial stage, the mice would struggle violently to try to escape this state, and after they could not break free, they showed an immobile state, which is considered a "despair" state. The immobility time of the tail - suspended mice in the tail - suspension test is used as an indicator to detect the despair behavior of animals and is often used in the primary screening test of antidepressant drugs.

In this experiment, among female animals, compared with the model control group, the immobility time of mice in the TM17 and TM20 groups was prolonged by more than 10%, but there was no statistical difference ($P > 0.05$), which might be due to the large individual differences among the animals in the group, resulting in no statistical significance; among male animals, the immobility time of mice in each sample group was prolonged by more than 10%. The immobility time of mice in the TM18 and TM20 groups was prolonged by about 20%, and there was a statistical difference ($P < 0.05$). It shows that after the TM series samples were continuously injected for 3 days, the sedative conditions of animals in each group had gender differences, and a stronger sedative effect could be shown on male mice.

After the preventive administration of the TM series samples, the duration of the immobile state of mice in the tail - suspension test is shown in Table 4.

Table 4 Duration of the Immobile State of Animals in the Tail - Suspension Test (n = 5)

| group | Average Duration of Immobile State (Mean ± Standard Deviation, Unit: Seconds) | | | |
|---|---|---|---|---|
| | female | *P* - value | male | *P* - value |
| model control group | 170.57±28.43 | -- | 151.23±35.02 | -- |
| TM17 | 198.88±15.08 | 0.060 | 177.67±20.05 | 0.054 |
| TM18 | 174.02±18.28 | 0.812 | 193.80±15.62 | 0.003** |
| TM19 | 161.06±30.02 | 0.513 | 177.36±16.49 | 0.057 |
| TM20 | 192.23±18.24 | 0.144 | 180.15±6.29 | 0.037* |
| Note: "*" indicates that compared with the model control group, P < 0.05; "**" indicates that compared with the model control group, P < 0.01. | | | | |

### 5. Experimental Conclusions

By constructing a model of high intracellular chloride ions and using the TM17 - TM20 series samples on the cell type with high intracellular chloride ions, through observation and analysis under a fluorescence microscope, it can be seen that the intracellular fluorescence intensity increased after the intervention of the TM series samples. This indicates that the TM17 - TM20 series samples can prolong the opening time of the chloride ion channel activated by GABA.

By preparing a mouse model of acute depression and using the TM17 - TM20 series samples for preventive administration, there was no significant difference in the immobility duration of female animals, while the immobility duration of male animals could be significantly prolonged. This shows that the TM17 - TM20 series samples have a sedative effect on mice with the acute depression model.

Combining the results of in vivo and in vitro experiments, it is speculated that the TM17 - TM20 series samples have the effect of treating depression.

## III. Patch Clamp Electrophysiological Experiment on GABAA Receptor (Primary Cells)

### 1. Liquids Used for Electrophysiological Recording

#### Extracellular Fluid (GABA-001-1)

140 mM NaCl, 5 mM CsCl, 2 mM $CaCl_2 \cdot 2H_2O$, 1 mM $MgCl_2 \cdot 6H_2O$, 5 mM HEPES, 10 mM D-Glucose, and the pH is adjusted to 7.4 with NaOH.

#### Intracellular Fluid (GABA-001-2)

130 mM CsCl, 0.1 mM $CaCl_2 \cdot 2H_2O$, 2 mM $MgCl_2 \cdot 6H_2O$, 1.1 mM EGTA, 5 mM $Na_2$-ATP, 10 mM HEPES, and the pH is adjusted to 7.2 with CsOH.

extracellular fluid can be stored for 2 weeks. After the intracellular fluid is prepared, it is aliquoted into tubes with 1 mL each and frozen in a -20 °C refrigerator. Freshly thawed intracellular fluid is used for daily experiments. All intracellular fluids should be used up within three months. If it exceeds three months, the old intracellular fluid should be discarded and newly prepared.

### 2. Patch Clamp Detection of Primary Hippocampal Neuronal Cells of SD Suckling Mice

The voltage stimulation protocol for whole-cell patch clamp recording of GABA receptor currents is as follows: After the whole-cell seal is formed, the cell membrane voltage is clamped at -70 mV. Record the GABA receptor current in the Gap-free mode (when detecting primary hippocampal neuronal cells of SD suckling mice, add 50 μM D-AP5, 20 μM DNQX, 10 μM Strychnine, and 300 nM TTX to the extracellular fluid to block NMDA, AMPA, Glycine, and Nav receptors). Record the peak currents after sequentially spraying low-concentration GABA (15 μM), a mixture of low-concentration GABA and the test substance at increasing concentrations (GABA 15 μM + TM17 0.1 μM, GABA 15 μM + TM17 1 μM), and 300 μM GABA onto the cell surface. The administration method of the test substance is that after each concentration of the test substance is administered 1 - 2 times, rinse with extracellular fluid for 2 - 3 minutes before detecting the next concentration. Finally, 300 μM GABA is given as a control. The experimental data are collected by the EPC-10 amplifier (HEKA) and stored in the PatchMaster (HEKA) software.

Use a microelectrode puller to pull capillary glass tubes into recording electrodes. Manipulate the microelectrode manipulator under an inverted microscope to make the recording electrode contact the cell, apply negative pressure suction to form a GΩ seal. After forming the GΩ seal, perform rapid capacitance compensation (pF), and then continue to apply negative pressure to break the cell membrane and form the whole-cell recording mode. Then perform slow capacitance compensation and record the membrane capacitance (pF) and series resistance. No leakage compensation is given.

Place the coverslip with cells on it in the recording bath of the inverted microscope. The working solutions of the test substances and the extracellular fluid without compounds are made to flow through the recording bath from low to high concentrations successively by gravity perfusion to act on the cells. Use a vacuum pump for liquid exchange during recording. All electrophysiological experiments are carried out at room temperature. Conduct three parallel experiments. The results are shown in Table 5 and Figures 1 - 3, where Figures 1, 2, and 3 respectively show the detection spectra of GABA 15 μM, GABA 15 μM + 0.1 μM TM 17, and GABA 15 μM + 1 μM TM 17.

Table 5: Detection Results of TM 17

| | Peak rHip GABAAR Current (pA) | | | | The percentage increase after calibration based on GABA 15μM | |
|---|---|---|---|---|---|---|
| cell number | GABA 15μM | GABA 15μM+ TM17 0.1μM | GABA 15μM+ TM 17 1μM | GABA 300 μM | GABA 15μM+ TM 17 0.1μM | GABA 15μM+ TM 17 1μM |
| S2-220628002 | -91.84 | -121.05 | -111.36 | -1027.90 | 131.81% | 121.26% |
| S2-220628005 | -271.00 | -712.41 | -744.97 | -1574.30 | 262.88% | 274.90% |
| S2-220628008 | -157.28 | -486.71 | -517.66 | -1077.90 | 309.45% | 329.13% |
| average | -173.37 | -440.06 | -458.00 | -1226.70 | 234.71% | 241.76% |
| standard deviation SD | 90.66 | 298.43 | 320.99 | 302.07 | 92.11% | 107.83% |

In the same manner as described above, TM18 is detected, and the results are shown in Table 6 below.

Table 6: Test results for TM 18

| | The percentage increase after calibration with GABA 15μM as the reference (TM18) | | |
|---|---|---|---|
| | GABA 15μM+TM 18 0.01μM | GABA 15μM+TM 18 0.1μM | GABA 15μM+TM 18 1μM |
| average | 129.85% | 288.44% | 108.82% |
| sample standard error (SE) | 18.76% | 83.55% | 21.76% |

The above experimental results show that under the action of TM17 and TM18, the inward current (chloride ion current) of the GABAA receptor increased significantly, indicating that TM17 and TM18 are excellent positive modulators of the GABAA receptor.

**IV. Patch Clamp Electrophysiological Experiment on GABAA Receptor (Stably Transfected Cells)**

**(I)** Stably Transfected Cell Lines and Cell Culture

1. The GABAA (α1β2γ2) cell line stably expressed by HEK293 cells was adopted, and its gene information includes GABA α1 (NP_000797.2), GABA β2 (NP_000804.1), and GABA γ2 (NP_000807.2).
2. Cell Preparation:

   1) Inoculate the cells in the culture medium in a 6-cm cell culture dish (containing 0.5 μg/mL puromycin and 10% fetal bovine serum). When the cell confluence is between 30% and 70%, remove the old culture medium, add 1 mL of PBS, gently rinse the cells, and then discard the PBS.
   2) Add 1 mL of TrypLeTM Express, gently shake the culture dish to make it cover the bottom of the cell culture dish. Then place it in a 37 °C carbon dioxide incubator for 2 - 3 minutes. After taking it out, use a pipette tip to gently blow and make the adherent cells suspended.
   3) Put the adherent cells into a sterile centrifuge tube and centrifuge at 1000 rpm/min for 3 minutes. Then adjust the cell concentration to $3 \times 10^4$ cells/mL. Take 500 μL of the cell suspension and inoculate it on the cell coverslips in a 24-well plate. After the cells adhere well, conduct electrophysiological experiments overnight.

**(II) Extracellular and Intracellular Fluids**

1) Extracellular Fluid (mM): 137 NaCl, 4 KCl, 1.8 $CaCl_2$, 1 $MgCl_2$, 10 Glucose, 10 HEPES. Adjust the pH to 7.4 with

NaOH.
2) Intracellular Fluid (mM): 140 CsCl, 5 EGTA, 10 HEPES. Adjust the pH to 7.2 with CsOH.

**(III) Preparation of Test Samples to be Measured**

1) Dilute the test samples into 30 mM stock solutions with dimethyl sulfoxide (DMSO).
2) Use DMSO to successively dilute the test sample stock solutions into 10 mM, 3 mM, 1 mM, 0.3 mM, and 0.1 mM secondary stock solutions.
3) Before the start of the experiment, take 5 μL each of the stock solution and the secondary stock solutions and dilute them into 5 mL of the extracellular fluid containing 3 μM GABA. After a thousand-fold dilution, the final concentrations of the test samples are 10 μM, 3 μM, 1 μM, 0.1 μM, and at these concentrations, they are used to detect the EC50 of the test samples by patch clamp.

**(IV) Whole-Cell Test Procedure**

1) Use the Sutter P-1000 microelectrode puller to pull glass recording electrodes.
2) Put the cell coverslip into the bath containing the extracellular fluid and observe the cells with a 10× objective lens.
3) Use the micromanipulator to make the glass electrode slowly approach the cell. When the tip of the glass electrode and the cell are close to the same horizontal plane in the field of view, adjust the objective lens to a 40× objective lens and slowly contact the cell surface.
4) When the glass electrode just contacts the cell surface, the access resistance slightly increases. Carefully continue to move the glass electrode downward until the access resistance increases to 1/3 - 2/3 of the initial access resistance, and then stop moving. Quickly and gently aspirate from the side hole of the holder to form a gigohm seal.
5) Slowly apply negative pressure to break the membrane and form the whole-cell recording mode.
6) Clamp the cell at -70 mV, record the peak GABA current, and use the gravity drug delivery and cumulative drug addition method to spray on the cell surface for 10 - 15 seconds. For the same cell, cumulatively add negative control (0.1% DMSO), GABA 3 μM, and a mixture of GABA 3 μM and the test sample (from low to high concentrations).

**(V) Data Processing**

1) Data Quality Control

The valid data in the experiment need to meet the following criteria:

Initial seal resistance > 1 GΩ; series resistance (Ra) < 15 MΩ; membrane resistance (Rm) > 200 MΩ.

2) Data Analysis

The data that meet the above GABA current quality standards are further analyzed according to the following steps:

2.1) Standardize the current of the test sample and the GABA EC10 (3 μM) current.

$$\text{Percentage increase in current (\%)} = \left(\frac{\text{Peak current of test sample}}{\text{Peak current of GABA EC10}} - 1\right)*100$$

2.2) Calculate the half - maximal activation concentration of each compound using the following equation:

$$Y=Bottom + (Top-Bottom)/(1+10^{\wedge}((LogEC50-X) \times HillSlope))$$

Use the above equation to perform nonlinear fitting on the dose-dependent effect. Among them, EC50 represents the half-maximal activation concentration, and Hillslope represents the Hill coefficient. Curve fitting and the calculation of EC50 are completed by using GraphPad Prism 5 software, and the results are shown in Figure 4 and Figure 5.

Compared with the EC50 of GABA as the control, which is 10.82 μM, for TM17, its EC50 is 8.708 μM; for TM18, its EC50 is 1.646 μM. This also indicates that under the action of TM17 and TM18, the inward current (chloride ion current) of the GABAA receptor increases significantly, once again confirming that TM17 and TM18 are excellent positive modulators of the GABAA receptor.

The above description is only the embodiments of the present invention and is not intended to limit the present invention. Any modification, equivalent replacement, or improvement made within the spirit and principle of the present invention shall be included within the protection scope of the present invention.

## Claims

1. A steroid compound or its pharmaceutically acceptable salt, **characterized in that** the steroid compound has a 9β,10α structure and has the following structural formula:

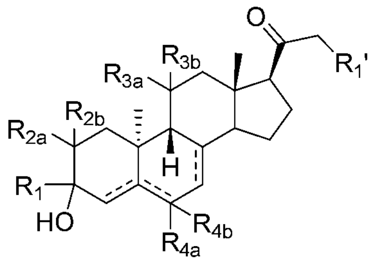

Formula I

wherein $R_1$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group, or $-CH_2OR_{1a}$, where $R_{1a}$ is selected from substituted or unsubstituted $C_{1\sim6}$ alkyl, or substituted or unsubstituted $C_{2\sim6}$ alkenyl;
wherein $R_{2a}$ and $R_{2b}$ are each independently selected from H, halogen, substituted or unsubstituted $C_{1\sim6}$ alkyl, substituted or unsubstituted $C_{2\sim6}$ alkenyl, substituted or unsubstituted $C_{2\sim6}$ alkynyl, substituted or unsubstituted $C_{3\sim6}$ carbocyclic group or $OR_{2c}$, where $R_{2c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group;
$R_{3a}$ and $R_{3b}$ are each independently selected from H or $OR_{3c}$, where $R_{3c}$ is selected from H, substituted or unsubstituted $C_{1\sim6}$ alkyl, substituted or unsubstituted $C_{2\sim6}$ alkenyl, substituted or unsubstituted $C_{2\sim6}$ alkynyl, or substituted or unsubstituted $C_{3\sim6}$ carbocyclic group, or $R_{3a}$ and $R_{3b}$ combine to form = O;
$R_{4a}$ and $R_{4b}$ are each independently selected from H, halogen, substituted or unsubstituted $C_{1\sim6}$ alkyl;
$R_1$' is selected from H, or substituted or unsubstituted heteroaryl;
\- - - - - - represents a single bond or a double bond;

when one of - - - - - - is a double bond, the adjacent - - - - - - is a single bond.

2. The steroid compound or its pharmaceutically acceptable salt according to claim 1, **characterized in that** the $R_1$ is selected from H, $C_1$~$_3$ alkyl, $C_1$~$_3$ haloalkyl or $-CH_2OR_{1a}$, wherein $R_{1a}$ is selected from $C_1$~$_3$ alkyl.

3. The steroid compound or its pharmaceutically acceptable salt according to claim 2, **characterized in that** the $R_1$ is selected from H.

4. The steroid compound or its pharmaceutically acceptable salt according to claim 2, **characterized in that** the $R_1$ is selected from methyl.

5. The steroid compound or its pharmaceutically acceptable salt according to claim 2, **characterized in that** the $R_1$ is selected from $-CH_2OCH_3$.

6. The steroid compound or its pharmaceutically acceptable salt according to claim 1, **characterized in that** the

configuration of $R_1$ is of the α-configuration or β-configuration, and correspondingly, the configuration of the -OH at the C-3 position is β-OH or α-OH.

7. The steroid compound or its pharmaceutically acceptable salt according to claim 6, **characterized in that** the configuration of $R_1$ is of the β-configuration and the C-3 position is α-OH.

8. The steroid compound or its pharmaceutically acceptable salt according to claim 1, **characterized in that** $R_{2a}$, $R_{2b}$, $R_{3a}$, $R_{3b}$, $R_{4a}$ and $R_{4b}$ are all H.

9. The steroid compound or its pharmaceutically acceptable salt according to claim 1, **characterized in that** ------ is a single bond, the C-8 position is β-H and the C-14 position is α-H.

10. The steroid compound or its pharmaceutically acceptable salt according to claim 1, **characterized in that** when $R_1$' is selected from substituted or unsubstituted heteroaryl, the heteroatom therein is N.

11. The steroid compound or its pharmaceutically acceptable salt according to any one of claims 1 to 10, **characterized in that** the steroid compound has the following structural formula:

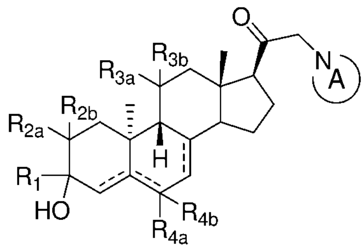

wherein ring A is a substituted or unsubstituted heteroaryl group, the heteroatom is N, and the number of heteroatom N is 1 - 4.

12. The steroid compound or its pharmaceutically acceptable salt according to claim 11, **characterized in that** the ring A is selected from the following groups:

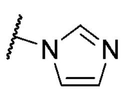 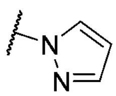 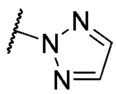 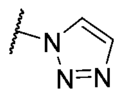

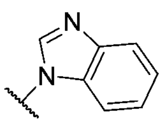 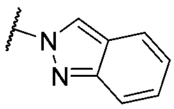 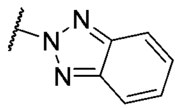 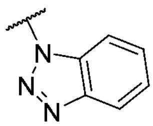

the above groups are either unsubstituted or substituted by groups selected from the following: H, halogen, $-NO_2$, -CN, -OR', $-N(R')_2$, -C(=O)R', -C(=O)OR', -OC(=O)R' or -OC(=O)OR', wherein R' is selected from H, substituted or unsubstituted $C_1$~$_6$ alkyl, substituted or unsubstituted $C_2$~$_6$ alkenyl, substituted or unsubstituted $C_2$~$_6$ alkynyl, substituted or unsubstituted $C_3$~$_6$ carbocyclic group, substituted or unsubstituted $C_3$~$_6$ heterocyclic group, substituted or unsubstituted aryl, or substituted or unsubstituted heteroaryl.

13. The steroid compound or its pharmaceutically acceptable salt according to claim 11, **characterized in that** the ring A is a five-membered ring, and the number of heteroatom N is 2 or 3.

14. The steroid compound or its pharmaceutically acceptable salt according to claim 11, **characterized in that** the steroid compound has the following structural formula:

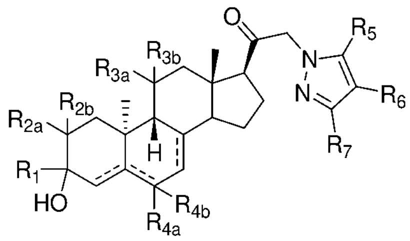

wherein $R_5$, $R_6$ and $R_7$ are each independently selected from H, halogen, $-NO_2$, -CN, -OR', $-N(R')_2$, -C(=O)R', -C(=O)OR', -OC(=O)R' or -OC(=O)OR', wherein R' is selected from H, substituted or unsubstituted $C_1$~$_6$ alkyl, substituted or unsubstituted $C_2$~$_6$ alkenyl, substituted or unsubstituted $C_2$~$_6$ alkynyl, substituted or unsubstituted $C_3$~$_6$ carbocyclic group, substituted or unsubstituted $C_3$~$_6$ heterocyclic group, substituted or unsubstituted aryl, or substituted or unsubstituted heteroaryl.

**15.** The steroid compound or its pharmaceutically acceptable salt according to claim 14, **characterized in that** one of the $R_5$, $R_6$ and $R_7$ is -CN and the rest are H.

**16.** The steroid compound or its pharmaceutically acceptable salt according to claim 15, **characterized in that** the $R_1$ is selected from H, $C_1$~$_3$ alkyl or $C_1$~$_3$ haloalkyl, and $R_{2a}$, $R_{2b}$, $R_{3a}$, $R_{3b}$, $R_{4a}$ and $R_{4b}$ are all H.

**17.** The steroid compound or its pharmaceutically acceptable salt according to claim 14, **characterized in that** the steroid compound is selected from the following structural formulas:

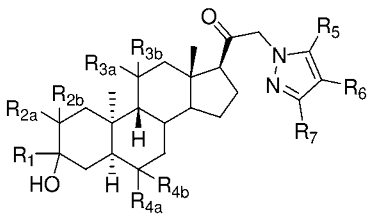
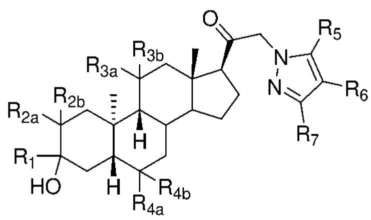
.

**18.** The steroid compound or its pharmaceutically acceptable salt according to claim 14, **characterized in that** the steroid compound is selected from the following structural formulas:

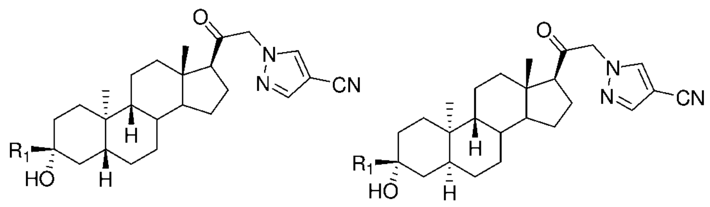
.

**19.** The steroid compound or its pharmaceutically acceptable salt according to claim 18, **characterized in that** the $R_1$ is selected from methyl or ethyl.

**20.** The steroid compound or its pharmaceutically acceptable salt according to claim 11, **characterized in that** the steroid compound has the following structural formula:

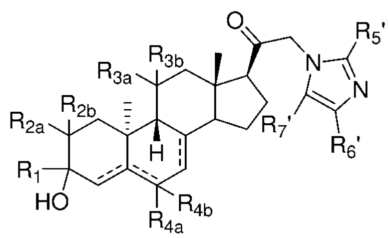

wherein $R_5'$, $R_6'$ and $R_7'$ are each independently selected from H, halogen, $-NO_2$, -CN, -OR', $-N(R')_2$, -C(=O)R', -C(=O)OR', -OC(=O)R' or -OC(=O)OR', wherein R' is selected from H, substituted or unsubstituted $C_1$~$_6$ alkyl, substituted or unsubstituted $C_2$~$_6$ alkenyl, substituted or unsubstituted $C_2$~$_6$ alkynyl, substituted or unsubstituted $C_3$~$_6$ carbocyclic group, substituted or unsubstituted $C_3$~$_6$ heterocyclic group, substituted or unsubstituted aryl, or substituted or unsubstituted heteroaryl.

**21.** The steroid compound or its pharmaceutically acceptable salt according to claim 20, **characterized in that** $R_5'$, $R_6'$ and $R_7'$ are all H.

**22.** The steroid compound or its pharmaceutically acceptable salt according to claim 20, **characterized in that** the $R_1$ is selected from H, $C_1$~$_3$ alkyl or $C_1$~$_3$ haloalkyl, and $R_{2a}$, $R_{2b}$, $R_{3a}$, $R_{3b}$, $R_{4a}$ and $R_{4b}$ are all H, and ------ is a single bond.

**23.** The steroid compound or its pharmaceutically acceptable salt according to claim 11, **characterized in that** the steroid compound is selected from the following structural formulas:

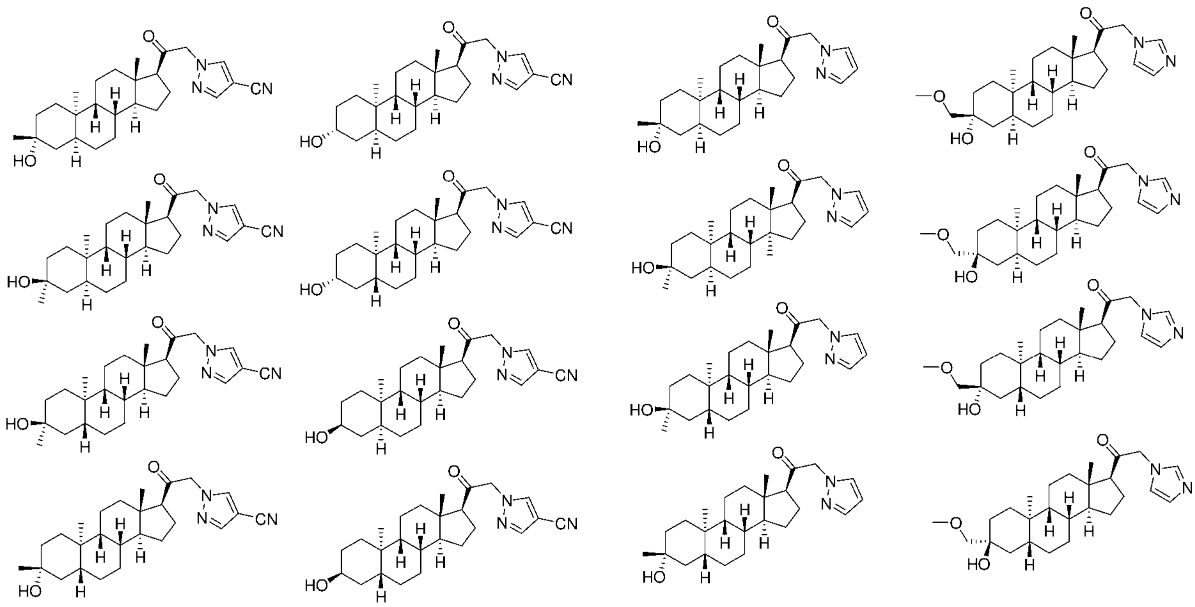

.

**24.** The steroid compound or its pharmaceutically acceptable salt according to any one of claims 1 to 10, **characterized in that** the steroid compound has the following structural formula:

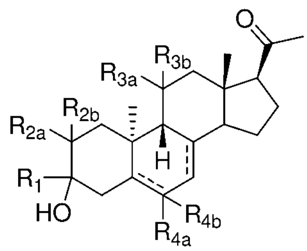

.

**25.** The steroid compound or its pharmaceutically acceptable salt according to claim 24, **characterized in that** the steroid compound has the following structural formula:

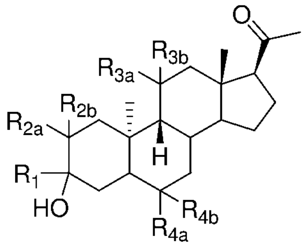

.

**26.** The steroid compound or its pharmaceutically acceptable salt according to claim 25, **characterized in that** the steroid compound is selected from the following structural formulas:

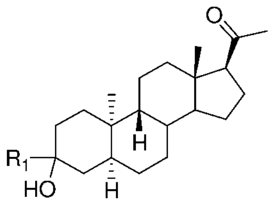

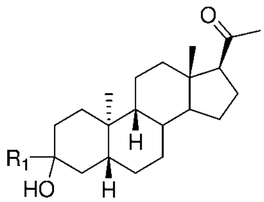

.

**27.** The steroid compound or its pharmaceutically acceptable salt according to any one of claims 1 to 10, **characterized in that** the steroid compound is selected from the following structural formulas:

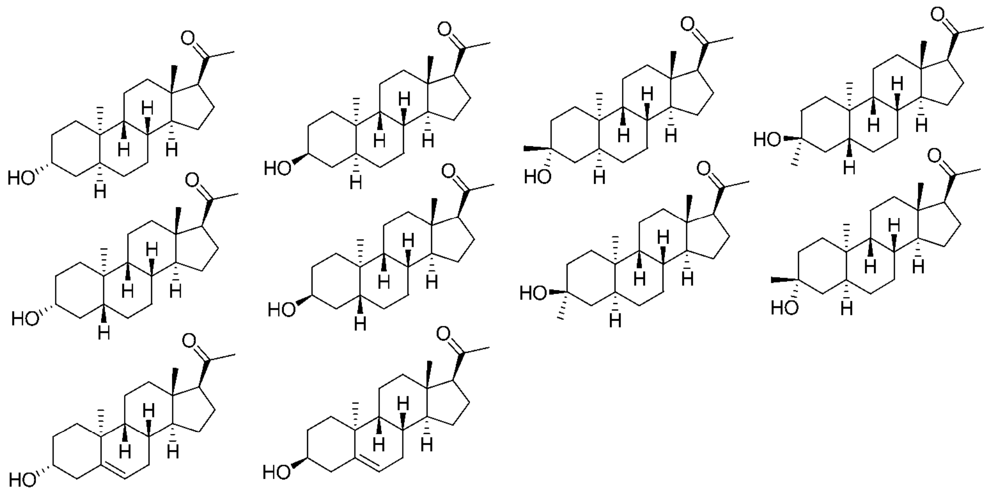

.

**28.** A method for preparing the steroid compound according to claim 11, **characterized in that** the steroid compound is prepared by a nucleophilic substitution reaction of a compound of the following formula:

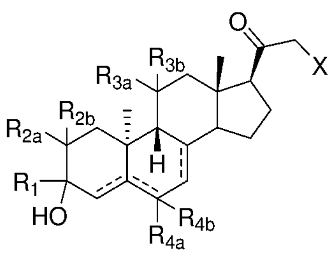

wherein X is halogen.

**29.** A method for preparing the steroid compound according to claim 24, **characterized in that** the steroid compound is prepared from dydrogesterone through a reduction reaction and/or an addition reaction.

**30.** An intermediate compound for preparing the steroid compound according to claim 11, **characterized in that** the intermediate compound has the following structural formula:

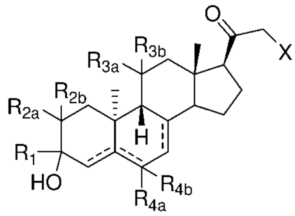

wherein X is halogen.

**31.** Application of the steroid compound or its pharmaceutically acceptable salt according to any one of claims 1 to 27 in the preparation of a medicament for treating disorders related to the central nervous system.

**32.** The application according to claim 31, wherein the disorders related to the central nervous system are selected from: sleep disorders, mood disorders, schizophrenia spectrum disorders, spastic disorders, memory disorders and/or cognitive disorders, movement disorders, personality disorders, autism spectrum disorders, pain, and traumatic brain injury.

**33.** The application according to claim 32, wherein the disorders related to the central nervous system are selected from: insomnia, depression, anxiety disorders, epilepsy, dementia, neuropathic pain or tremors.

**34.** The application according to claim 33, the dementia is Alzheimer's disease.

**35.** A pharmaceutical composition comprising the steroid compound or its pharmaceutically acceptable salt according to any one of claims 1 to 27, and pharmaceutically acceptable excipients.

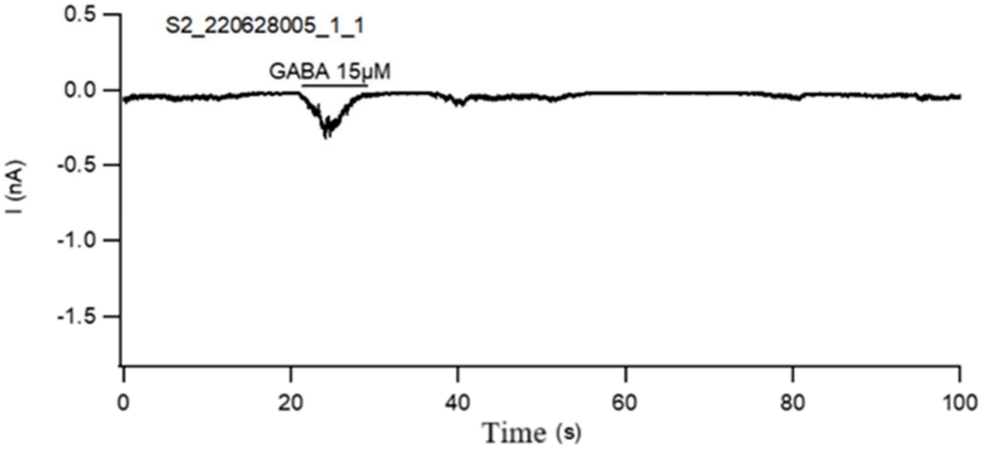
S2_220628005_1_1
GABA 15μM
I (nA)
Time (s)

Fig. 1

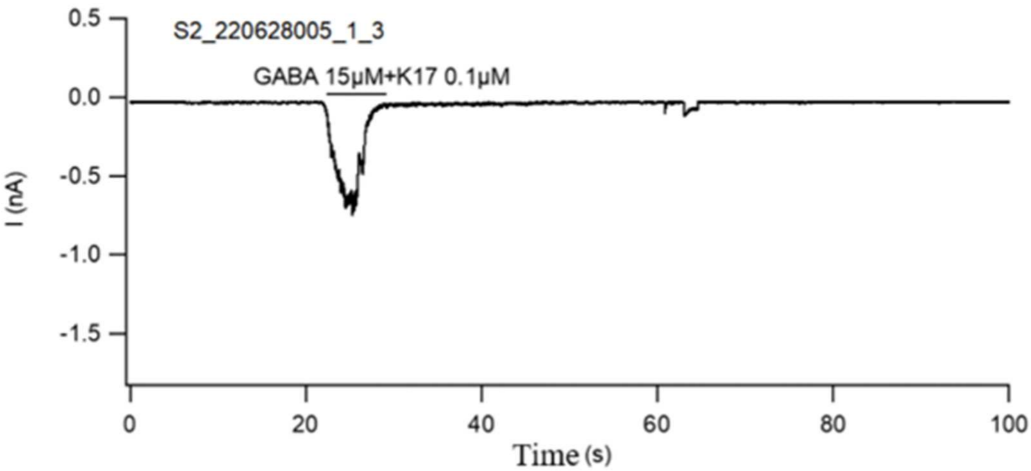
S2_220628005_1_3
GABA 15μM+K17 0.1μM
I (nA)
Time (s)

Fig. 2

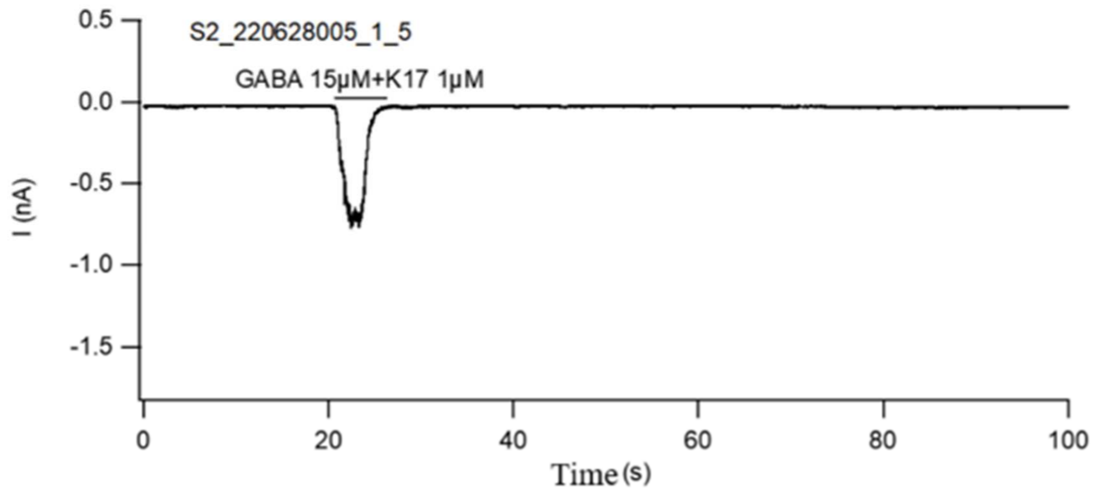
S2_220628005_1_5
GABA 15μM+K17 1μM
0.5
0.0
-0.5
-1.0
-1.5
I (nA)
0
20
40
60
80
100
Time (s)

Fig. 3

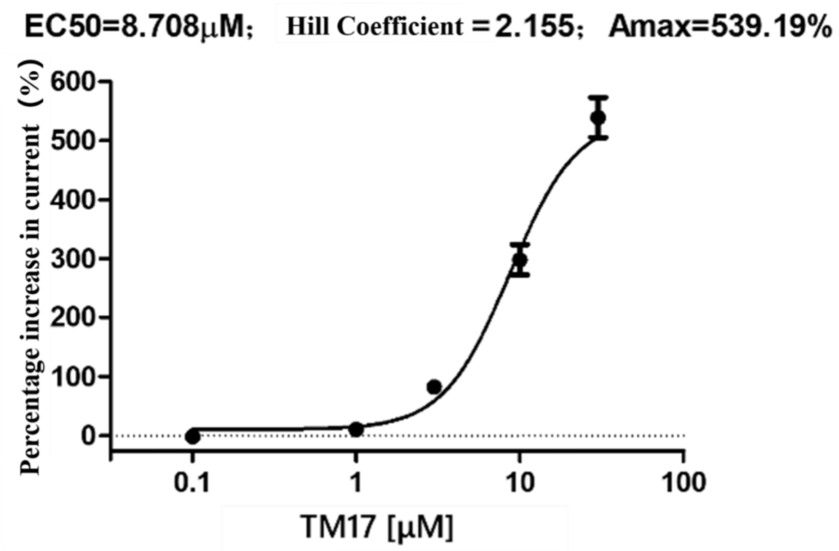
EC50=8.708μM; Hill Coefficient = 2.155; Amax=539.19%
600
500
400
300
200
100
0
Percentage increase in current (%)
0.1
1
10
100
TM17 [μM]

Fig. 4

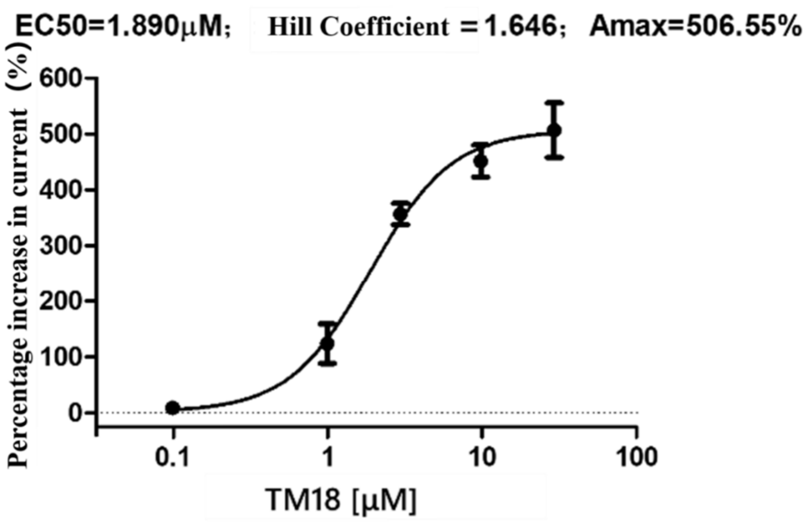
EC50=1.890μM; Hill Coefficient = 1.646; Amax=506.55%
Percentage increase in current (%)
600
500
400
300
200
100
0
0.1
1
10
100
TM18 [μM]

Fig. 5

**INTERNATIONAL SEARCH REPORT**

International application No.
**PCT/CN2022/108717**

**A. CLASSIFICATION OF SUBJECT MATTER**

C07J 43/00(2006.01)i; C07J 7/00(2006.01)i; A61K 31/57(2006.01)i; A61P 25/28(2006.01)i

According to International Patent Classification (IPC) or to both national classification and IPC

**B. FIELDS SEARCHED**

Minimum documentation searched (classification system followed by classification symbols)

C07J; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 甾体, 氨基丁酸, 别孕烷醇酮, 中枢神经, 上海醇健, Steroids, GABA, aminobutyric acid, brexanolone, allopregnanolone, central nervous system, CNS, SHANGHAI STEROL INDUSTRIAL

**C. DOCUMENTS CONSIDERED TO BE RELEVANT**

| Category* | Citation of document, with indication, where appropriate, of the relevant passages | Relevant to claim No. |
|---|---|---|
| X | "RN 4243-94-1"<br>*STN Registry*, 16 November 1984 (1984-11-16),<br>p. 1 | 1-3, 8, 24-26 |
| X | WO 2022040545 A1 (INTRA CELLULAR THERAPIES, INC.) 24 February 2022 (2022-02-24)<br>claims 1, 14 and 20-22, and description, p. 30 | 1-23, 28, 30-35 |
| X | WO 2015134670 A1 (ZHANG, MingBao) 11 September 2015 (2015-09-11)<br>claims 11-15, and description, p. 14 | 1-23, 28, 30-35 |
| A | CN 111171101 A (NINGBO DONGLONG OPTOELECTRONIC SCIENCE & TECHNOLOGY CO., LTD. et al.) 19 May 2020 (2020-05-19)<br>description, paragraph [0021] | 1-35 |
| A | WO 2009108388 A2 (UNIVERSITY OF TENNESSEE RESEARCH FOUNDATION) 03 September 2009 (2009-09-03)<br>figure 1 | 1-35 |
| A | WO 2020185710 A1 (ALAIRION, INC.) 17 September 2020 (2020-09-17)<br>abstract, and claims | 1-92 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 December 2022** | **21 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/108717**

**C. DOCUMENTS CONSIDERED TO BE RELEVANT**

| Category* | Citation of document, with indication, where appropriate, of the relevant passages | Relevant to claim No. |
|---|---|---|
| A | WO 2021067089 A1 (ATHENEN THERAPEUTICS, INC.) 08 April 2021 (2021-04-08) abstract, and claims 1-10 | 1-35 |
| A | WO 2021195301 A1 (SAGE THERAPEUTICS, INC.) 30 September 2021 (2021-09-30) abstract, and claims 1-75 | 1-35 |

Form PCT/ISA/210 (second sheet) (January 2015)

**INTERNATIONAL SEARCH REPORT**
**Information on patent family members**

International application No.
**PCT/CN2022/108717**

| Patent document cited in search report | Publication date (day/month/year) | Patent family member(s) | Publication date (day/month/year) |
|---|---|---|---|
| WO 2022040545 A1 | 24 February 2022 | None | |
| WO 2015134670 A1 | 11 September 2015 | None | |
| CN 111171101 A | 19 May 2020 | None | |
| WO 2009108388 A2 | 03 September 2009 | US 2014200201 A1 | 17 July 2014 |
| | | US 2011118228 A1 | 19 May 2011 |
| WO 2020185710 A1 | 17 September 2020 | None | |
| WO 2021067089 A1 | 08 April 2021 | IL 291835 A | 01 June 2022 |
| | | KR 20220103707 A | 22 July 2022 |
| | | CN 115087450 A | 20 September 2022 |
| | | CA 3159087 A1 | 08 April 2021 |
| WO 2021195301 A1 | 30 September 2021 | CO 2022013777 A2 | 11 October 2022 |
| | | CA 3176854 A1 | 30 September 2021 |
| | | TW 202143977 A | 01 December 2021 |
| | | AU 2021241622 A1 | 20 October 2022 |
| | | WO 2021195297 A1 | 30 September 2021 |
| | | TW 202202146 A | 16 January 2022 |
| | | IL 296645 A | 01 November 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **THEODORA W. GREENE** ; **PETER G. M. WUTS**. Protective Groups in Organic Synthesis. Wiley - Interscience, 2007